# EUROPEAN PATENT APPLICATION

(11) **EP 4 768 068 A1**
(43) Date of publication of application: **01.07.2026**
(21) Application number: 24884219.7
(22) Date of filing: 02.09.2024
(51) Int. Cl.: A61M 60/178, A61M 60/857, A61M 60/174, A61M 25/01

(54) **BLOOD PUMP**

(30) Priority: 31.10.2023 CN 202311432166; 31.10.2023 CN 202311447400
(71) Applicant: Shenzhen Core Medical Technology Co., Ltd., Shenzhen, Guangdong 518000 (CN)
(72) Inventor: XIE, Duanqing, Shenzhen, Guangdong 518000 (CN); YU, Shunzhou, Shenzhen, Guangdong 518000 (CN); DENG, Dazhao, Shenzhen, Guangdong 518000 (CN); HUANG, Jiaming, Shenzhen, Guangdong 518000 (CN)
(74) Representative: Appleyard Lees IP LLP
(86) International application number: PCT/CN2024/116341
(87) International publication number: WO 2025/092212

(57) **Abstract**

A blood pump (10), comprising a sleeve housing (100), a driving unit (200), and a sleeve assembly (300). The driving unit (200) is arranged in the sleeve housing (100). The sleeve housing (100) and the driving unit (200) are spaced by a flow gap (500). The sleeve assembly (300) comprises a compensation tube (310), the compensation tube (310) being fixedly connected to the sleeve housing (100) and being in communication with the flow gap (500) to form a blood flow channel. A proximal end of the sleeve housing (100) is provided with a first opening (111), and a distal end of the sleeve assembly is provided with a second opening (321).

## Description

This application claims priority to Chinese Patent Application No. CN202311447400.2, entitled "Blood Pump", and Chinese Patent Application No. CN202311432166.6, entitled "Blood Pump", filed with China National Intellectual Property Administration on October 31, 2023, the entire contents of which are incorporated herein by reference.

### TECHNICAL FIELD

The present application relates to the field of medical instrument technologies, and in particular, to a blood pump.

### BACKGROUND

In the related art, a blood pump capable of intervening in the right ventricle has been developed. The blood pump can pass through the pulmonary valve from the right ventricle to enter the pulmonary artery, so as to assist the right ventricle in pumping blood. In order to adapt to a delivery path of the right ventricle, an axial length of a pump body of the blood pump is designed to be relatively short. However, the pump body of the blood pump is required to pass through the pulmonary valve and be secured by the pulmonary valve via clamping. As the pulmonary valve opens and closes alternately with pulsation of the heart, the excessively short axial length of the pump body of the blood pump makes it prone to be axially displaced by the pulmonary valve during alternating opening and closing of the pulmonary valve, so that the blood pump has the risk of dislodging from the pulmonary valve.

The information disclosed above in the BACKGROUND of the present application is only used for understanding the background of the concept of the present application, and may contain information that does not constitute the prior art.

### SUMMARY

Based on this, the present application provides a blood pump which can be applied to assist the right ventricle in pumping blood from the right ventricle into the pulmonary artery and reduce the risk of the blood pump becoming dislodged from the pulmonary valve.

The present application provides a blood pump, including a housing, a drive unit and a cannula assembly. The drive unit is arranged inside the housing. A proximal end of the housing is fixedly connected to a proximal end of the drive unit or a catheter of the blood pump, and an inner peripheral surface of the housing is spaced apart from the drive unit to form a flow passage gap. The cannula assembly includes a compensation tube. The compensation tube is elastic, and the compensation tube is fixedly connected to a distal end of the housing to be in communication with the flow passage gap to form a blood flow channel. The proximal end of the housing is provided with a first opening, a distal end of the cannula assembly is provided with a second opening, and the second opening is in communication with the first opening via the blood flow channel.

The present application further provides a blood pump, including:
a housing, the housing being provided with a liquid inlet and a liquid outlet;
a drive unit arranged inside the housing, a proximal end of the housing being fixedly connected to a proximal end of the drive unit or a catheter of the blood pump, an inner peripheral surface of the housing being spaced apart from the drive unit to form a blood flow channel, and the blood flow channel being in communication with the liquid inlet and the liquid outlet, wherein the drive unit includes a motor and an impeller connected to the motor;
a cannula assembly, the cannula assembly being provided with a liquid discharge port, the cannula assembly including a compensation tube, the compensation tube being elastic, and the compensation tube being fixedly connected to and in communication with a distal end of the housing, such that the liquid discharge port is in communication with the liquid outlet of the housing through an inner cavity of the compensation tube; and
a flow straightening device, the flow straightening device being arranged in the blood flow channel, and the flow straightening device being capable of guiding blood to flow from an outer periphery of the motor to the impeller.

Details of one or more embodiments of the present application are set forth in the accompanying drawings and the description below. Other features, objects, and advantages of the present application will become more apparent from the description, the accompanying drawings, and the claims.

### BRIEF DESCRIPTION OF THE DRAWINGS

In order to illustrate the technical solutions in the embodiments of the present application more clearly, the drawings required for describing the embodiments or the prior art will be described briefly. Apparently, the following described drawings are merely for some embodiments of the present application, and other drawings can be derived from these drawings by those of ordinary skill in the art without any creative effort.
FIG. 1 is a schematic diagram showing an anatomical structure of a human heart.
FIG. 2 is a schematic diagram showing a blood pump according to an embodiment of the present application being applied to assist the right ventricle in pumping blood.
FIG. 3 is a schematic diagram showing blood flow during systole after the blood pump according to an embodiment of the present application is implanted into the heart.
FIG. 4 is a schematic diagram showing blood flow during diastole after the blood pump shown in FIG. 3 is implanted into the heart.
FIG. 5 is a front view of a blood pump according to an embodiment of the present application.
FIG. 6 is a sectional view of the blood pump shown in FIG. 5 taken along line A-A.
FIG. 7 is a partial enlarged view of a portion B in the blood pump shown in FIG. 6.
FIG. 8 is a schematic exploded diagram of the blood pump shown in FIG. 5.
FIG. 9 is a further schematic exploded diagram of the blood pump shown in FIG. 8.
FIG. 10 is an axial perspective view of a compensation tube of the blood pump shown in FIG. 6.
FIG. 11 is an axial perspective view of a connection tube of the blood pump shown in FIG. 6.
FIG. 12 is an axial perspective view of an outlet tube of the blood pump shown in FIG. 6.
FIG. 13 is an axial perspective view of an inlet tube of the blood pump shown in FIG. 6.
FIG. 14 is an axial perspective view of a housing and a catheter of the blood pump shown in FIG. 6.
FIG. 15 is a schematic structural diagram of a blood pump according to another embodiment of the present application.
FIG. 16 is a schematic diagram showing an internal structure of the blood pump shown in FIG. 15.
FIG. 17 is a partial enlarged view of a portion C of the blood pump shown in FIG. 16.
FIG. 18 is a partial enlarged view of a portion E of the blood pump shown in FIG. 17.
FIG. 19 is an assembly view of a catheter, a drive unit and a flow straightening device of the blood pump shown in FIG. 16.
FIG. 20 is an assembly view of the catheter and a motor of the blood pump shown in FIG. 19.
FIG. 21 is a partial structural view showing the assembly of the catheter and the motor of the blood pump shown in FIG. 20.
FIG. 22 is a schematic structural diagram of a flow straightening device of a blood pump according to an embodiment of the present application.
FIG. 23 is a schematic diagram of a flow straightening principle of the flow straightening device shown in FIG. 22.

### DETAILED DESCRIPTION

In order to make the objectives, technical solutions and advantages of the present application more clearly understood, the present application will be further described in detail with the accompanying drawings and embodiments. It should be understood that the specific embodiments described herein are only used to explain the present application and not to limit the present application.

It should be noted that when an element is referred to as being "fixed on" or "arranged on" another element, the element may be directly or indirectly located on the other element. When an element is referred to as being "connected to" another element, the element may be directly or indirectly connected to the other element.

In addition, the terms such as "first" and "second" are used herein for purposes of description and are not intended to indicate or imply relative importance or implicitly indicate the quantity of the technical features indicated. Thus, the feature defined with "first" and "second" may explicitly or implicitly include at least one of these features. In the description of the present application, "a plurality of" means two or more, unless otherwise clearly and specifically defined.

To illustrate the technical solution of the present application, the following description is provided with reference to the specific accompanying drawings and embodiments.

In the present application, "proximal end" is defined as the end close to an operator, and "distal end" is defined as the end away from the operator.

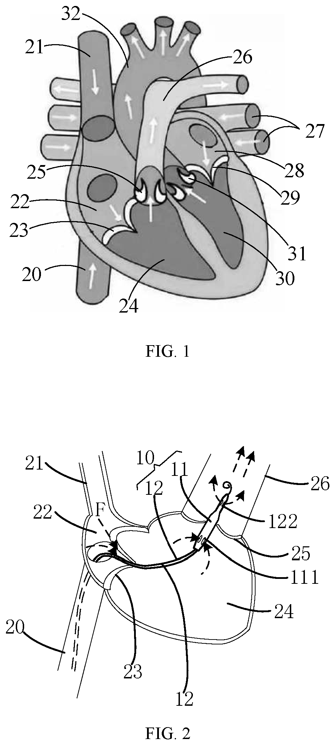

Referring to FIG. 1, FIG. 1 is a schematic diagram showing an anatomical structure of a human heart. In the related art, an interventional assist device, also referred to as a blood pump, is mainly applied to be inserted into a ventricle through a patient's blood vessel to assist the ventricle in pumping blood. In order to facilitate understanding of the application of the blood pump, the heart structure and the blood flow direction are briefly described below. The blood circulation in the human body includes systemic circulation and pulmonary circulation. The blood is ejected from the left ventricle 30 into the aorta 32 through the aortic valve 31, and then flows to capillaries throughout the body through the aorta 32 for material exchange, converting arterial blood into venous blood. The venous blood then flows back to the right atrium 22 via the superior vena cava 21 and the inferior vena cava 20. This cycle is referred to as the systemic circulation. Subsequently, the blood in the right atrium 22 enters the right ventricle 24 through the tricuspid valve 23, is then ejected from the right ventricle 24 into the pulmonary artery 26 through the pulmonary valve 25, and then flows to various pulmonary capillaries through the pulmonary artery 26 for gas exchange, converting the venous blood into the arterial blood. The arterial blood finally flows back to the left atrium 28 via the pulmonary vein 27, and the blood in the left atrium 28 enters the left ventricle 30 through the mitral valve 29. This cycle is referred to as the pulmonary circulation.

A common type of blood pump is a left ventricular assist pump. Such a left ventricular assist pump typically has a relatively long pump body. When the left ventricular assist pump is applied to the left ventricle 30, the left ventricular assist pump is usually inserted from the aorta 32 through the aortic valve 31, such that a blood inlet of the left ventricular assist pump extends into the left ventricle 30, while a blood outlet of the left ventricular assist pump is positioned within the aorta 32, i.e., only a distal portion of the left ventricular assist pump extends into the left ventricle 30. Thus, the left ventricular assist pump can assist the left ventricle 30 in pumping the blood from the left ventricle 30 into the aorta 32. Since a path for the left ventricular assist pump to pass from an ascending portion of the aorta 32 into the left ventricle 30 through the aortic valve 31 is relatively aligned in a same axial direction, the left ventricular assist pump with the longer pump body can be adapted to a delivery path of the left ventricle 30.

If the right ventricle 24 of the patient has functional impairment, a blood pump is also required to assist the right ventricle 24 in pumping blood. Accordingly, efforts have been made to apply the left ventricular assist pump to assist the right ventricle 24 in pumping the blood. However, a delivery path for implanting a blood pump into the right ventricle is generally from the inferior vena cava 20 or the superior vena cava 21 to the right atrium, the tricuspid valve 23, the right ventricle 24, the pulmonary valve 25, and finally to the pulmonary artery 26. It can be seen that the delivery path for implanting the blood pump into the right ventricle involves multiple curves, a short path, and a complex internal structure. Since the pump body of the conventional left ventricular assist pump has an excessively long axial length and cannot easily pass through the delivery path of the right ventricle 24, such left ventricular assist pumps are difficult to apply to the right ventricle 24.

Therefore, a right ventricular assist pump has emerged in the market. A pump body of this right ventricular assist pump is designed with a relatively short axial length, so that the right ventricular assist pump can pass from the right ventricle 24 through the pulmonary valve 25 into the pulmonary artery 26 to assist the right ventricle 24 in pumping the blood. However, the pump body of the right ventricular assist pump needs to pass through the pulmonary valve 25 and be secured by the pulmonary valve 25 via clamping. As the pulmonary valve 25 alternately opens and closes with pulsation of the heart, the excessively short axial length of the pump body of the blood pump makes it prone to be axially displaced by the pulmonary valve 25 during alternating opening and closing of the pulmonary valve, so that the blood pump has the risk of dislodging from the pulmonary valve 25.

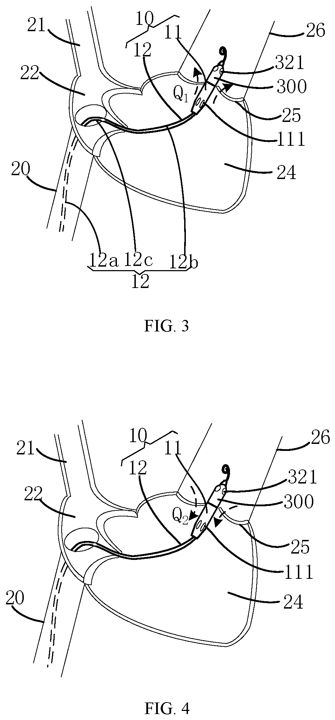

Referring to FIG. 3 and FIG. 4, to address the above problem, the present application provides a blood pump 10. The blood pump 10 is primarily intended for use as a right ventricular assist pump. The blood pump 10 is configured to be inserted from the right ventricle 24 into the pulmonary artery 26 to assist the right ventricle 24 in pumping blood into the pulmonary artery 26, thereby achieving pressure relief for the right ventricle 24 and reducing the risk of the blood pump becoming dislodged from the pulmonary valve 25. Certainly, in other embodiments, the blood pump 10 may be used as a left ventricular assist pump. It should be noted here that in the field of medical instrument technologies, the end of a medical device closer to a physician or operator is generally referred to as the proximal end, while the end farther from the physician or operator is referred to as the distal end.

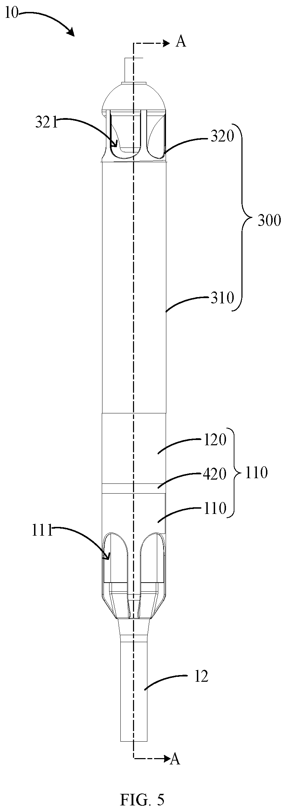

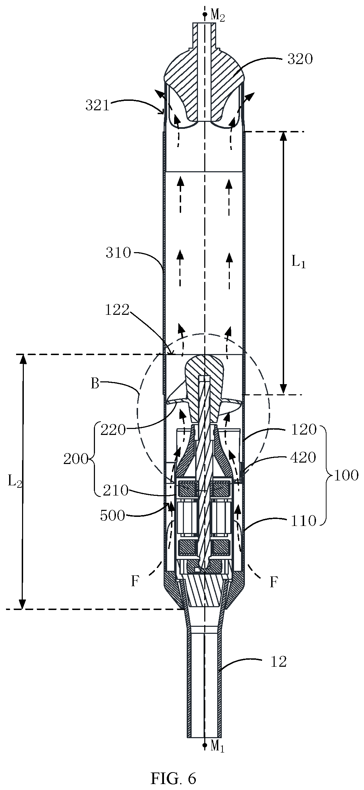

Referring to FIG. 5 and FIG. 6, in an embodiment of the blood pump according to the present application, the blood pump 10 includes a housing 100, a drive unit 200 and a cannula assembly 300. The drive unit 200 is configured to drive blood to flow, and the drive unit 200 is arranged inside the housing 100. A proximal end of the housing 100 is fixedly connected to a proximal end of the drive unit 200 or a catheter 12 of the blood pump 10, and an inner surface of the housing 100 is spaced apart from the drive unit 200 to form a flow passage gap 500. The cannula assembly 300 includes a compensation tube 310, and the compensation tube 310 is fixedly connected to a distal end of the housing 100 and in communication with the flow passage gap 500 to form a blood flow channel. The proximal end of the housing 100 is provided with a first opening 111, a distal end of the cannula assembly 300 is provided with a second opening 321, and the second opening 321 is in communication with the first opening 111 via the blood flow channel.

Referring to FIG. 6, one of the first opening 111 and the second opening 321 may serve as a blood inlet, and the other may serve as a blood outlet. When the blood pump 10 is used as the right ventricular assist pump, the first opening 111 is used as the blood inlet, and the second opening 321 is used as the blood outlet; after the blood pump 10 is started, the drive unit 200 drives the blood F to enter the flow passage gap 500 from the first opening 111, and the blood F then enters the compensation tube 310 from the flow passage gap 500, and finally is discharged from the second opening 321 to assist pressure relief of the right ventricle 24, so as to reduce excessive dilation during blood filling of the right ventricle 24.

In other embodiments, the blood pump 10 may be used as the left ventricular assist pump. When the blood pump 10 is used as the left ventricular assist pump, the first opening 111 serves as the blood outlet and the second opening 321 serves as the blood inlet. After the blood pump 10 is started, the drive unit 200 drives the blood to enter the compensation tube 310 from the second opening 321, and the blood then enters the flow passage gap 500 from the compensation tube 310, and finally is discharged from the first opening 111.

It may be understood that, in the blood pump 10, the housing 100 and the drive unit 200 constitute a pump body 11 of the blood pump 10 and serve as a hard part. Since the drive unit 200 is arranged inside the housing 100, an axial length of the pump body 11 is reduced. When the blood pump 10 is implanted, the compensation tube 310 of the blood pump 10 enters the right ventricle 24 before the pump body 11, and the compensation tube 310 can elastically deform in the right ventricle 24, so that the pump body 11 with the shorter axial length can also enter the right ventricle 24 after the compensation tube 310, and then, the pump body 11 of the blood pump 10 and the compensation tube 310 thereof can deflect upwards in the right ventricle 24 to pass through the pulmonary valve 25 into the pulmonary artery 26. Since the compensation tube 310 appropriately compensates for the shortened axial length of the pump body 11, not only the pump body 11 can be clamped by the pulmonary valve 25, but also the compensation tube 310 can be clamped by the pulmonary valve 25, so that an axial length of the blood pump 10 for clamping and positioning by the pulmonary valve 25 can be effectively increased. Therefore, after the blood pump 10 is arranged through the pulmonary valve 25, the pump body 11 and the compensation tube 310 of the blood pump 10 can be clamped by the pulmonary valve 25, so as to reduce the risk that the pump body 11 falls off from the pulmonary valve 25.

Specifically, as shown in FIG. 3, one delivery path of the blood pump 10 is as follows: the pump body 11 of the blood pump 10 passes from the inferior vena cava 20 into the right atrium 22, then passes from the right atrium 22 through the tricuspid valve 23 into the right ventricle 24, and then passes from the right ventricle 24 through the pulmonary valve 25 to partially extend into the pulmonary artery 26, so as to ensure that a liquid inlet 1240 of the blood pump 10 can be located in the right ventricle 24, and a liquid outlet 220a of the blood pump 10 can be located in the pulmonary artery 26. Another delivery path of the blood pump 10 is as follows: the pump body 11 of the blood pump 10 enters the right atrium 22 from the superior vena cava 21, then passes from the right atrium 22 through the tricuspid valve 23 into the right ventricle 24, and then passes from the right ventricle 24 through the pulmonary valve 25 to partially extend into the pulmonary artery 26, so as to ensure that the liquid inlet 1240 of the blood pump 10 can be located in the right ventricle 24, and the liquid outlet 220a of the blood pump 10 can be located in the pulmonary artery 26.

As shown in FIG. 1 and FIG. 3, the delivery path for pushing the blood pump 10 from the inferior vena cava 20 or the superior vena cava 21 to the pulmonary artery 26 is short, and has multiple bends and complex internal tissue. Since the axial length of the pump body 11 of the blood pump 10 is short and the compensation tube 310 thereof can be elastically deformed, the pump body 11 can pass through the bends of the short delivery path and turn (or deflect) upwards in the narrow right ventricle 24 to enter the pulmonary artery 26. During the process that the pump body 11 turns upwards from the right ventricle 24 into the pulmonary artery 26, the compensation tube 310 enters the right ventricle 24 before the pump body 11 and is elastically deformed in the right ventricle 24, so that the pump body 11 can successively enter the right ventricle 24 and turn (or deflect) upwards from the right ventricle 24 to partially extend into the pulmonary artery 26, thereby ensuring that the first opening 111 of the blood pump 10 can be located in the right ventricle 24, and the second opening 321 of the blood pump 10 can be located in the pulmonary artery 26. Therefore, difficulty generated when the blood pump 10 enters the right ventricle 24 can be greatly lowered, and smoothness of passing of the blood pump 10 through the delivery path can be improved.

It may be readily understood that the heart 20 always performs contraction and dilation movement. As shown in FIG. 3, during systole of the heart 20, the pulmonary artery 26 opens, allowing blood to flow from the right ventricle 24 into the pulmonary artery 26, and in this case, a clamping force applied by the pulmonary valve 25 to the blood pump 10 is decreased, so that the pump body 11 of the blood pump 10 has a tendency to move towards the interior of the pulmonary artery 26 under an impact of the blood flowing in a direction Q₁. Similarly, as shown in FIG. 4, when the heart 20 dilates, the pulmonary valve 25 is closed, and the pulmonary valve 25 has a tendency to move the pump body 11 of the blood pump 10 towards the interior of the right ventricle 24 along a direction Q₂.

Therefore, in the blood pump 10 according to the present application, the axial length of the pump body 11 is shortened to be approximately equal to an axial length of the housing 100 by arranging the drive unit 200 inside the housing 100, so that after the compensation tube 310 of the blood pump 10 enters the right ventricle 24, the pump body 11 with the short length can enter the right ventricle 24 with the compensation tube 310 only through small elastic deformation of the compensation tube 310. Due to the short axial length of the pump body 11 and the elastic deformation of the compensation tube 310, the pump body 11 of the blood pump 10 can deflect upwards in the right ventricle and then pass through the pulmonary valve 25 into the pulmonary artery 26, so that the first opening 111 can be located in the right ventricle 24, and the second opening 321 of the blood pump 10 can be located in the pulmonary artery 26. Moreover, since the compensation tube 310 and the pump body 11 are axially arranged, not only the pump body 11 can be clamped by the pulmonary valve 25, but also the compensation tube 310 can be clamped by the pulmonary valve 25, so that the axial length of the blood pump 10 for clamping and positioning by the pulmonary valve can be effectively increased. Therefore, after the blood pump 10 passes through the pulmonary valve 25, the pulmonary valve 25 can clamp at least one of the pump body 11 or the compensation tube 310 during the process that the pulmonary valve 25 alternately opens and closes with the pulsation of the heart, so that the risk of the blood pump 10 becoming dislodged from the pulmonary valve 25 can be reduced.

In an embodiment, the first opening 111 and the second opening 321 of the blood pump 10 can both be in communication with the blood flow channel, i.e., either of the first opening 111 and the second opening 321 can be used as the blood inlet, and the other can be used as the blood outlet. In the present application, the opening as the blood inlet is not limited, but for the convenience of understanding and description, the following embodiments take the first opening 111 as the blood inlet and the second opening 321 as the blood outlet as an example to illustrate specific implementations of the blood pump 10 according to the present application. It should be understood that the reverse condition is similar and therefore not repeated.

Referring to FIG. 3, in an embodiment, the blood pump 10 can pass through the inferior vena cava 20, the right atrium 22, the right ventricle 24 and the pulmonary valve 25 into the pulmonary artery 26, so that the first opening 111 is located in the right ventricle 24 and the second opening 321 is located in the pulmonary artery 26. Thus, during working, the blood pump 10 can pump the blood directly into the pulmonary artery 26, so as to reduce the condition that excessive accumulation of the blood in the right ventricle 24 results in excessive dilation of the right ventricle 24. Thus, safety of use of the blood pump 10 is improved. Moreover, since the first opening 111 is located in the right ventricle 24, the blood pump 10 can guide the blood in the superior vena cava 21 and the inferior vena cava 20 to flow into the right ventricle 24 through the right atrium 22 during working, so as to assist the heart 20 in conveying the blood. A blood flow path and direction within the blood pump 10 are shown by the dashed arrows F in FIG. 6.

Referring to FIG. 5 to FIG. 6, for the conventional left ventricle assist pump, an elastic cannula is usually connected to a distal end of the pump body 11, and only a part of the elastic cannula extends into the left ventricle, so that in order to adapt to the left ventricular delivery path, a length of the elastic cannula is several times the length of the pump body 11, the elastic cannula is usually pre-modeled in a bent shape, and therefore, the elastic cannula can be elastically deformed in a curving direction thereof. In an embodiment of the present application, the compensation tube 310 is configured to appropriately compensate for the shortened length of the pump body 11 after the drive unit 200 is received in the housing 100, so that an axial length of the compensation tube 310 is designed to be short to ensure that after the whole compensation tube 310 enters the right ventricle 24, the pump body 11 of the blood pump 10 can also enter the right ventricle 24. Optionally, the compensation tube 310 has a first axial length L₁. The first axial length L₁ is greater than or equal to 20mm and less than or equal to 35mm, i.e., 20mm≤L₁≤35mm. For example, the first axial length L₁ may be, but is not limited to, 22mm, 25mm, 28mm, 30mm, 34mm, or the like.

Further, since the compensation tube 310 has a relatively short axial length and does not require significant directional bending during implantation into the right ventricle, the compensation tube 310 is configured as a straight tube extending in an axial direction of the drive unit 200 in this embodiment. Such a configuration eliminates the need for a bending and shaping process step for the compensation tube 310 of the present application, thus reducing the manufacturing complexity and improving the production efficiency. Since the compensation tube 310 is a straight tube, the compensation tube 310 has a linear central axis M₁M₂. Optionally, the central axes of the compensation tube 310, the housing 100 and the drive unit 200 are located on the same straight line. The compensation tube 310, the housing 100 and the drive unit 200 share the same central axis M₁M₂. In this way, the blood flow channel formed by the communication between the flow passage gap 500 and the compensation tube 310 extends linearly in the axial direction, resulting in less resistance to blood flow through the blood flow channel, thereby allowing the blood to pass through the blood flow channel more smoothly.

Referring to FIG. 6, in an embodiment, an outer peripheral surface of the compensation tube 310 and an outer peripheral surface of the housing 100 are configured to be aligned in an axial direction of the compensation tube 310, so that an overall shape of the blood pump 10 can be smooth and regular, thereby improving smoothness of the implantation process of the blood pump 10, reducing obstruction of flow of the blood outside the blood pump 10, and also reducing a probability of thrombus formation caused by blood residing on a surface of the blood pump 10.

Referring to FIG. 6, in an embodiment, since an inner peripheral surface of the compensation tube 310 and an inner peripheral surface of the housing 100 both form a side wall of the blood flow channel, the inner peripheral surface of the compensation tube 310 and the inner peripheral surface of the housing 100 are configured to be aligned in the axial direction of the compensation tube 310, so as to avoid forming a variable diameter step at connection between the compensation tube 310 and the housing 100, so that the side wall of the blood flow channel becomes smoother in the axial direction, and thus, the resistance of the blood flow channel to the blood flow can be reduced, and the blood can pass through the blood flow channel more smoothly, thus improving a blood pumping efficiency of the blood pump 10.

Referring to FIG. 6, in an embodiment, the compensation tube 310 has a first axial length L₁, the housing 100 has a second axial length L₂, and a ratio of the first axial length L₁ to the second axial length L₂ is greater than or equal to 0.7 and less than or equal to 1.3, i.e., 0.7≤L₁/L₂≤1.3, i.e., 0.7L₂≤L₁≤1.3L₂. Since the housing 100 and the drive unit 200 constitute the hard part of the pump body 11 of the blood pump 10, the compensation tube 310 constitutes an elastic part, and an axial length of the hard part of the pump body 11 is substantially equal to the length of the housing 100, the ratio of the first axial length L₁ of the compensation tube 310 to the second axial length L₂ of the housing 100 is defined to be 0.7≤L₁/L₂≤1.3, which equivalently defines a size ratio of the elastic part to the hard part of the blood pump 10 to be 0.7≤L₁/L₂≤1.3. Thus, the length of the compensation tube 310 can be prevented from being overlong, and difficulty generated when the pump body 11 enters the right ventricle is reduced; a size of the hard part of the pump body 11 is not too short, and the axial length of the drive unit 200 of the pump body 11 is ensured not to be shortened excessively, so that the drive unit 200 has a sufficient driving force.

In an embodiment, the compensation tube 310 may be configured as a multi-layer composite structure to balance flexible deformability, support capability, and toughness. For example, the compensation tube 310 includes an inner membrane, an outer membrane, and an elastic coil arranged between the inner membrane and the outer membrane, so as to form the compensation tube 310 that achieves a balance between elastic deformability and connection support capability. It will be readily understood that the inner membrane is attached to an inner side of the elastic coil to form an inner peripheral wall for blood flow, and the outer membrane is attached to an outer side of the elastic coil to form an outer peripheral wall of the compensation tube 310. Certainly, the compensation tube 310 is not limited to be of the three-layer structure, and a number of layers and the materials used for the layered structure of the compensation tube 310 may be adaptively adjusted according to actual requirements. For example, a braided layer may be further incorporated into the compensation tube 310 to improve the toughness.

In an embodiment, the housing 100 is of a non-elastic structure. Optionally, the housing 100 is made of a hard material. Thus, the housing 100 can have high lightness, so that the structurally stable flow passage gap 500 can be constructed between the housing 100 and the drive unit 200 to stably deliver the blood. For example, the hard material may be a metal material, a polymer material, or the like.

Referring to FIG. 6 to FIG. 7, in an embodiment, the drive unit 200 includes a motor 210 and an impeller 220 connected to the motor 210. Both the motor 210 and the impeller 220 are received in the housing 100, and the motor 210 can drive the impeller 220 to rotate, so as to drive the blood to flow. In this regard, it is considered that since the first axial length L₁ of the compensation tube 310 is relatively short, when the blood pump 10 passes through the right atrium, the right ventricle and the pulmonary valve in sequence into the pulmonary artery along the delivery path of the right ventricular, the blood pump 10 may bend at a proximal end of the compensation tube 310 due to the narrow delivery path. If the impeller 220 extends from the interior of the housing 100 into the interior of the compensation tube 310, when the proximal end of the compensation tube 310 undergoes bending deformation, the inner peripheral surface of the compensation tube 310 may contact or abut against the impeller 220, thus interfering with the impeller 220 and hindering the rotation of the impeller 220.

In view of this, optionally, the housing 100 has a distal tube port 122 in communication with the compensation tube 310. A distal end of the impeller 220 has a distalmost point 223 farthest from the motor 210, and the distalmost point 223 is located within the housing 100 and does not extend beyond the distal tube port 122, such that the impeller 220 does not extend into the compensation tube 310. Specifically, since the housing 100 has a relatively rigid structure, a spatially stable inner cavity can be provided to facilitate the stable rotation of the impeller 220. When the blood pump 10 is inserted into the pulmonary artery along the delivery path of the right ventricular, even if the blood pump 10 bends at the proximal end of the compensation tube 310, the inner peripheral surface of the compensation tube 310 does not contact the impeller 220 because the impeller 220 does not extend into the compensation tube 310, thereby not interfering with the rotation of the impeller 220.

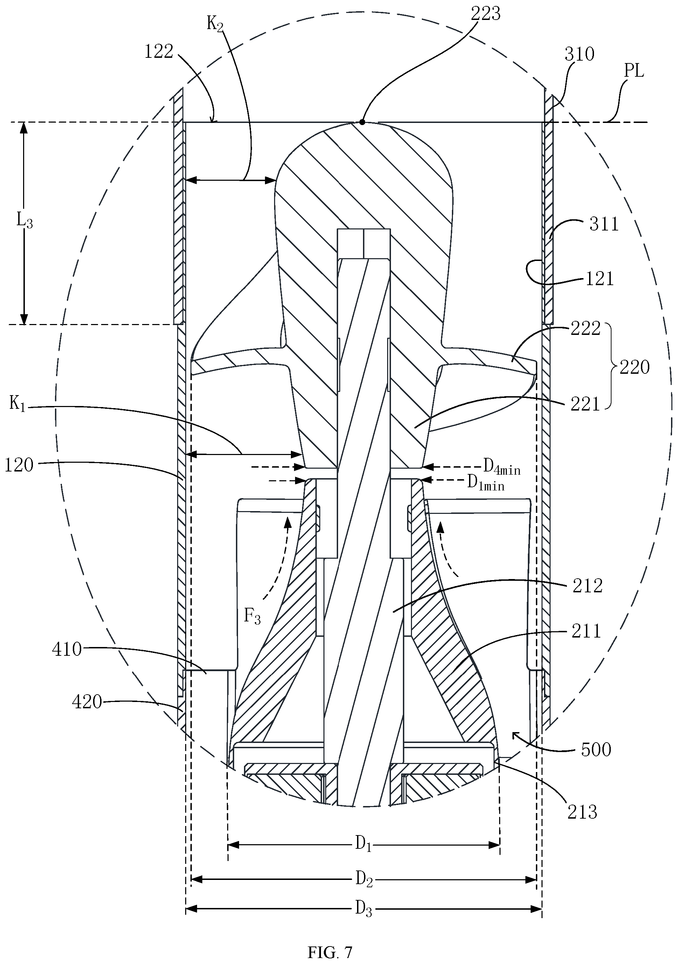

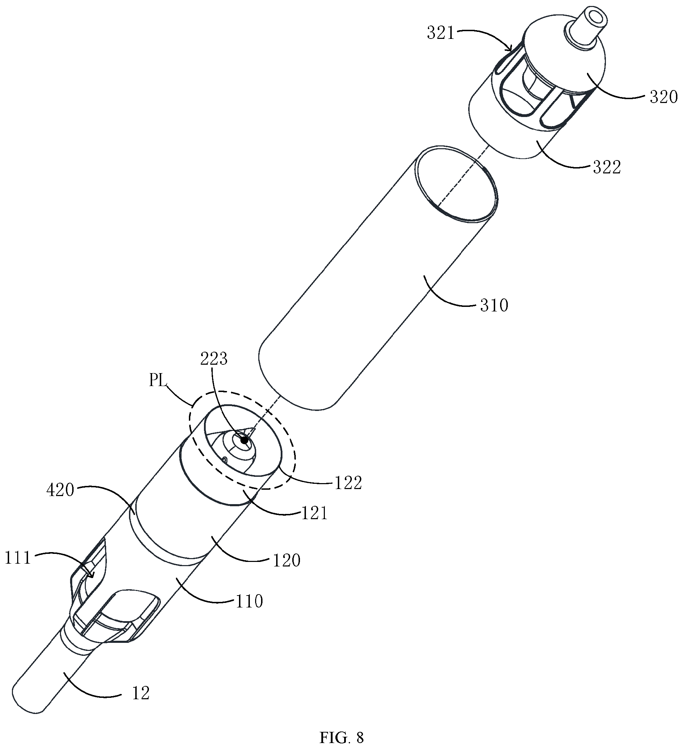

Referring to FIG. 7 and FIG. 8, in an embodiment, the distal point 223 of the impeller 220 and the distal tube port 122 are both located on a first plane PL. The first plane PL is a plane perpendicular to an axis of the drive unit, i.e., the distal end of the impeller 220 extends to the distal tube port 122 of the housing 100 to fully utilize an axial space of the housing 100 to arrange the long impeller 220, so that the impeller 220 can have a larger size, thereby driving more blood to flow and increasing a pumped blood flow rate. Certainly, in other embodiments, the distal point 223 of the impeller 220 may be axially spaced apart from the distal tube port 122.

Referring to FIG. 7, FIG. 9 and FIG. 10, in an embodiment, the distal end of the housing 100 is provided with a first insertion portion 121, and the proximal end of the compensation tube 310 is provided with a second insertion portion 311. The second insertion portion 311 is sleeved over an outer peripheral surface of the first insertion portion 121 and is adhesively secured to the outer peripheral surface of the first insertion portion 121.

Referring to FIG. 6 and FIG. 7, optionally, an overlapping region of the first insertion portion 121 and the second insertion portion 311 has a third axial length L₃. A ratio of the third axial length L₃ to the first axial length L₁ of the compensation tube 310 is greater than or equal to 0.1 and less than or equal to 0.3, i.e., 0.1≤L₃/L₁≤0.3, i.e., 0.1L₁≤L₃≤0.3L₁. L₃ may be, but is not limited to, 0.12L₁, 0.15L₁, 0.2L₁, 0.25L₁, and 0.3L₁.

Specifically, due to the short length of the compensation tube 310, when the compensation tube 310 is bent and deformed, the proximal end of the compensation tube 310 is subjected to a large bending force, and connection between the second insertion portion 311 and the first insertion portion 121 is subjected to a large bending force. By defining the ratio of the third axial length L₃ of the overlapping region of the first insertion portion 121 and the second insertion portion 311 to the first axial length L₁ of the compensation tube 310 to be 0.1≤L₃/L₁≤0.3, the second insertion portion 311 can have a larger area in sleeving fit with the first insertion portion 121, so that the second insertion portion 311 is more firmly connected to the first insertion portion 121. Thus, when the compensation tube 310 undergoes bending deformation, even if a relatively large bending force is applied to the proximal end of the compensation tube 310, the connection between the second insertion portion 311 and the first insertion portion 121 is not prone to loosening or detachment, thereby effectively improving the safety performance of the blood pump 10, i.e., by reasonably setting the third axial length L₃, the stability of the connection between the housing 100 and the compensation tube 310 is enhanced.

Referring to FIG. 7, for the conventional blood pump, the motor thereof is usually directly connected to the cannula assembly, only the impeller is arranged inside the cannula assembly, an outer diameter of the motor is usually consistent with an outer diameter of the cannula assembly, and therefore, a diameter of the impeller is usually smaller than that of the motor. In the present application, the drive unit 200 of the blood pump 10 is arranged in the housing 100, i.e., both the motor 210 and the impeller 220 are arranged in the housing 100. Assuming that the diameter of the motor 210 is a first diameter D₁, the diameter of the impeller 220 is a second diameter D₂, and an inner diameter of the housing 100 is a third diameter D₃, the third diameter should be greater than both the first diameter and the second diameter, i.e., D₃>D₁ and D₃>D₂.

In view of this, the impeller 220 can be designed to be large by making full use of the inner space of the housing 100. Therefore, optionally, a relationship among the first diameter D₁ of the motor 210, the second diameter D₂ of the impeller 220 and the third diameter D₃ of the housing 100 is as follows: the third diameter is greater than the second diameter, and the second diameter is greater than the first diameter, i.e., D₃>D₂>D₁, i.e., compared to the conventional blood pump, the diameter of the impeller 220 of the blood pump 10 according to the present application can be designed to be larger than the diameter of the motor 210, so that the impeller 220 can have a larger radial size than the motor 210, and thus, output power of the impeller 220 can be increased to improve the overall blood pumping efficiency of the blood pump 10. It may be understood that the diameter D₁ of the motor 210 is a maximum radial size of the motor 2210, and the diameter D₂ of the impeller 220 is also a maximum radial size of the impeller 220; the inner diameter D₃ of the housing 100 is an inner diameter of a region of the housing 100 receiving the motor 210 and the impeller 220.

Referring to FIG. 7, in an embodiment, the impeller 220 includes a hub 221 and blades 222, and the blades 222 are arranged on an outer periphery of the hub 221. The hub 221 and a shaft support base 211 are opposed to each other in the axial direction of the impeller 220. Since the impeller 220 is an axial-flow impeller, the blood driven by the impeller 220 possesses not only axial kinetic energy but also a portion of radial kinetic energy. The axial kinetic energy enables the blood to rapidly flow through an inner cavity of the compensation tube 310 in the axial direction of the compensation tube 310.

Based on this, in order to enable the blood to obtain large axial kinetic energy after the blood passes through the impeller 220, in this embodiment, a diameter of the hub 221 is gradually increased in a direction from the motor 210 to the impeller 220, so that a distance between an outer peripheral surface of the hub 221 and the inner peripheral surface of the housing 100 is gradually decreased in a diameter expansion direction of the hub 221. The diameter expansion direction is a direction from the motor 210 to the impeller 220. Such a configuration, a distal end of the hub 221 can have a large diameter, so that a gap K₂ between an outer peripheral surface of the distal end of the hub 221 and the inner peripheral surface of the housing 100 is smaller than a gap K₁ between an outer peripheral surface of a proximal end of the hub 221 and the inner peripheral surface of the housing 100, i.e., K₂<K₁. Blood F₃ enters the impeller 220 from the gap K₁ and is driven to rotate spirally by the impeller 220, so as to be gradually pushed by the impeller 220 towards the gap K₂. Since a path from the gap K₁ to the gap K₂ gradually becomes narrower, the blood F₃ is gradually compressed spirally in this process, so that radial potential energy obtained when the blood F₃ is driven to spiral by the impeller 220 is converted into axial potential energy, the axial potential energy of the blood F₃ is increased, an axial flowing component velocity of the blood F₃ can be increased, and therefore, the blood F₃ rapidly flows through the inner cavity of the compensation tube 310 in the axial direction of the compensation tube 310.

Referring to FIG. 6 and FIG. 7, in an embodiment, the motor 210 includes a motor body 213 and the shaft support base 211, and the shaft support base 211 is connected to a distal end of the motor body 213; a motor shaft 212 of the motor 210 passes through the shaft support base 211 from the motor body 213 to be fixedly connected to the impeller 220. Optionally, a distal end of the shaft support base 211 is axially opposite to the proximal end of the hub 221; a diameter of the shaft support base 211 gradually decreases in the direction from the motor 210 to the impeller 220. In this way, the blood on an outer periphery of the shaft support base 211 can be guided by an outer peripheral surface of the shaft support base 211 to flow in a diameter-reducing direction thereof, so that the blood is guided by the shaft support base 211 to flow into the impeller 220, so as to increase a flow rate of the blood driven by the impeller 220.

Further, the shaft support base 211 has a first minimum outer diameter D₁ₘᵢₙ at its distal end, the hub 221 has a second minimum outer diameter D₄ₘᵢₙ at its proximal end, and the second minimum outer diameter D₄ₘᵢₙ is smaller than or equal to the first minimum outer diameter D₁ₘᵢₙ, i.e., D₄ₘᵢₙ≤D₁ₘᵢₙ. It is easily understood that the hub 221 has a minimum radial size at the proximal end thereof, the shaft support base 211 has a minimum radial size at the distal end thereof, and the proximal end of the hub 221 is opposite and adjacent to the distal end of the shaft support base 211 in the axial direction of the impeller 220. Since the second minimum outer diameter D₄ₘᵢₙ is smaller than or equal to the first minimum outer diameter D₁ₘᵢₙ, when the blood is guided by the outer peripheral surface of the shaft support base 211 to flow towards the impeller 220 in the diameter-reducing direction thereof, the blood separated from the outer peripheral surface of the distal end of the shaft support base 211 can flow to the outer peripheral surface of the hub 221 of the impeller 220, so that the blood is prevented from colliding with a surface of the proximal end of the hub 221, and loss of the kinetic energy of the blood is reduced.

In other words, since in a region where the shaft support base 211 and the hub 221 are close to each other, a radial dimension of the shaft support base 211 is greater than or equal to that of the hub 221, the shaft support base 211 can guide the blood to smoothly across the transition between the shaft support base 211 and the hub 221. Therefore, in one aspect, the smoothness of the blood flow is improved, allowing the blood pump 10 to have higher pumping efficiency. In the other aspect, the risk of blood remaining at the transition between the shaft support base 211 and the hub 221 and forming thrombus can be reduced.

Referring to FIG. 7 to FIG. 9, in an embodiment, the housing 100 includes an inlet tube 110, a fixing ring 420 and a connection tube 120. The inlet tube 110 and the connection tube 120 are connected to two ends of the fixing ring 420 respectively. The inlet tube 110 is provided with the first opening 111. An end of the inlet tube 110 away from the fixing ring 420 (i.e., a proximal end of the inlet tube 110) is fixedly connected to the catheter 12 or the proximal end of the drive unit 200. An end of the connection tube 120 away from the fixing ring 420 (i.e., a distal end of the connection tube 120) is connected to the compensation tube 310. An inner wall surface of the fixing ring 420 is fixedly connected to an outer peripheral surface of the motor 210 through a support member 410.

Specifically, the fixing ring 420 is sleeved over an outer periphery of the motor 210, and the inner wall surface of the fixing ring 420 is spaced apart from the outer periphery surface of the motor 210; the inner wall surface of the fixing ring 420 is arranged on the plurality of support members 410, and the plurality of support members 410 are fixedly connected to the outer peripheral surface of the motor 210 to support and fix the fixing ring 420. The fixing ring 420 forms a side wall of a middle portion of the housing 100. Therefore, the fixing ring 420 is stably supported by the support member 410, and the fixing ring 420 achieves a central support effect on the housing 100, so that the inlet tube 110 and the connection tube 120 can have stable relative positions relative to an outer surface of the motor 210, so as to improve stability of the structure of the blood flow channel constructed between the housing 100 and the drive unit 200.

It should be noted that a shape and a structure of the support member 410 are not particularly limited. The support member 410 may be a support rib extending in a radial direction of the motor 210, or a deflector extending in an axial direction of the motor 210. During assembly, the inlet tube 110 of the housing 100 can be fitted over an outer periphery of a proximal end of the motor 210 from the proximal end of the motor 210 and fixedly connected to a proximal end of the fixing ring 420; and the connection tube 120 of the housing 100 is fitted over an outer periphery of a distal end of the motor 210 from the distal end of the motor 210 and fixedly connected to a distal end of the fixing ring 420, so that the housing 100 and the motor 210 are fixedly connected, thus improving assembly convenience.

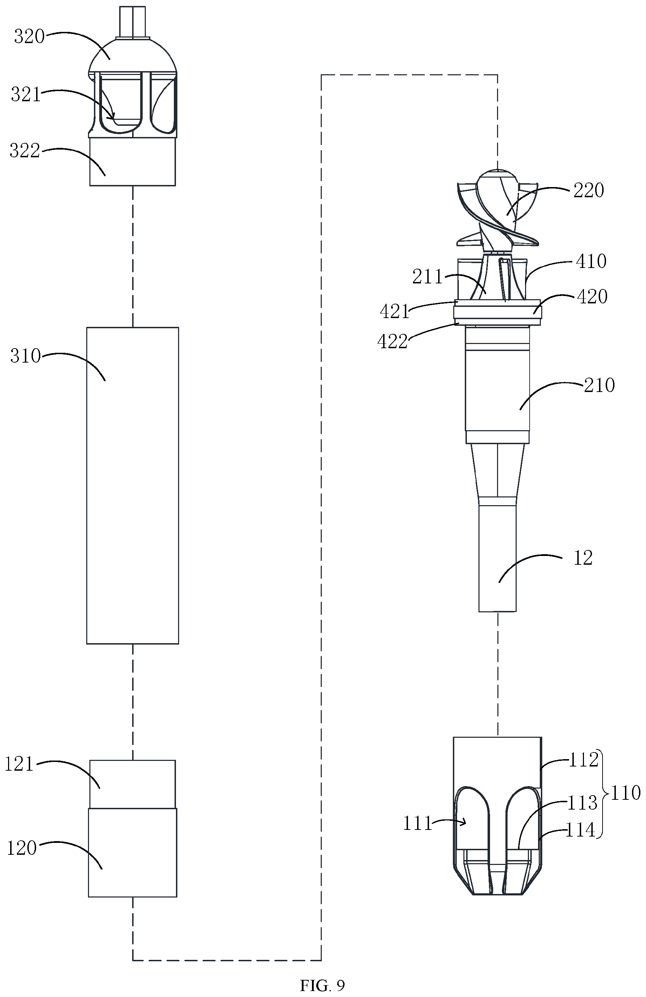

Referring to FIG. 9, FIG. 11 and FIG. 12, optionally, the first insertion portion 121 is arranged at the distal end of the connection tube 120, and the connection tube 120 is engaged with the second insertion portion 311 of the compensation tube 310 in an interference fit via the first insertion portion 121.

Referring to FIG. 9, FIG. 12 and FIG. 13, the distal end of the fixing ring 420 is provided with a seventh insertion portion 421 and an eighth insertion portion 422, an end of the connection tube 120 away from the compensation tube 310 is provided with a fifth insertion portion 123, and the seventh insertion portion 421 and the fifth insertion portion 123 are in insertion fit. The inlet tube 110 is provided with a sixth insertion portion 116, and the eighth insertion portion 422 is engaged with the sixth insertion portion 116 in an interference fit. Such a configuration, the fixing ring 420 is inserted into the connection tube 120 and the inlet tube 110 respectively, so as to form the housing 100.

Referring to FIG. 9, FIG. 13 and FIG. 14, in an embodiment, the inlet tube 110 includes a straight tube 112, a diameter-variable tube 113, and a plurality of connection arms 114 connecting the straight tube 112 and the diameter-variable tube 113. The straight tube 112 is connected to the fixing ring 420. The diameter-variable tube 113 is sleeved over an outer periphery of a connection between the catheter 12 and the drive unit 200, and is fixedly connected to at least one of the drive unit 200 or the catheter 12. The plurality of connection arms 114 are spaced apart in a circumferential direction of the straight tube 112, and one first opening 111 is formed between each two adjacent connection arms 114.

Specifically, the straight tube 112 is configured as a straight tube shape, so that an inner peripheral surface of the straight tube 112 forms a cylindrical surface, reducing fluid resistance in the blood flow channel. The diameter-variable tube 113 is configured in a conical shape. By sleeving the diameter-variable tube 113 over the outer periphery of the connection between the catheter 12 and the drive unit 200, the connection can be encapsulated within the diameter-variable tube 113, preventing adhesive at the connection from being washed away by blood. The diameter-variable tube 113 has two ports, one of which is in interference fit with the outer peripheral surface of the distal end of the catheter 12, and the other of which is in interference fit with the outer peripheral surface of the proximal end of the drive unit 200. The straight tube 112, the diameter-variable tube 113, and the connection arms 114 may be integrally formed.

Further, inner wall surfaces of the straight tube 112, the fixing ring 420 and the connection tube 120 are aligned in the axial direction to facilitate smooth blood flow. Moreover, outer wall surfaces of the straight tube 112, the fixing ring 420 and the connection tube 120 are also aligned in the axial direction. Such a configuration, the straight tube 112, the fixing ring 420 and the connection tube 120 together form a regular cylindrical body. Such a configuration, in one aspect, a smooth and regular outer peripheral surface of the housing 100 is provided, thus reducing damage to human tissue during the implantation process of the blood pump 10. In the other aspect, a smooth and regular inner peripheral surface of the housing 100 is also provided, thus reducing the fluid resistance in the blood flow channel, minimizing the loss of the kinetic energy of the blood flowing in the blood flow channel, improving the blood pumping efficiency, and reducing damage to blood cells.

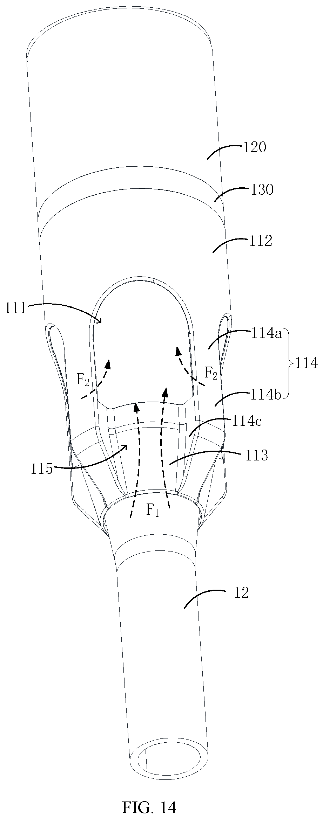

Referring to FIG. 14, in an embodiment, each connection arm 114 includes a first connection portion 114a connected to the straight tube 112, and a second connection portion 114b connecting the first connection portion 114a and the diameter-variable tube 113. The connection arms 114 are located on an outer peripheral surface of the diameter-variable tube 113. Each two adjacent second connection portions 114b are spaced apart to form a notch groove 115, and the notch groove 115 is in communication with a proximal end of the first opening 111 in an axial direction of the inlet tube 110.

When the drive unit 200 operates to generate a suction force, blood F₂ is radially sucked from a periphery of the inlet tube 110 by the first opening 111, and then continues to be driven by the drive unit 200 to switch to axially flow into the flow passage gap 500; blood F₁ can axially reach an inner side of the first opening 111 from the notch groove 115, and push the blood F₂ entering from the first opening 111 to axially flow, so as to push the blood F₂ to flow into the flow passage gap 500 at an increased speed, thereby effectively increasing the flow rate of the blood entering from the first opening 111 and improving the blood pumping efficiency of the blood pump 10, i.e., the second connection portion 114 cooperates with the diameter-variable tube 113 to form the notch groove 115, so that smoothness of passing of the blood into the first opening 111 can be improved, so as to lower a probability of blood congestion at the first opening 111.

Referring to FIG. 14, further, a flow guide surface 114c is arranged on a side of the second connection portion 114b facing the notch groove 115, and the flow guide surface 114c is configured to guide the blood to axially reach the inner side of the first opening 111 from the notch groove 115, so as to accelerate blood flowing and improve the blood pumping efficiency. The flow guide surface 114c is substantially prismatic, and a distal portion of the flow guide surface 114c extends to a side of a proximal end of the first connection portion 114a.

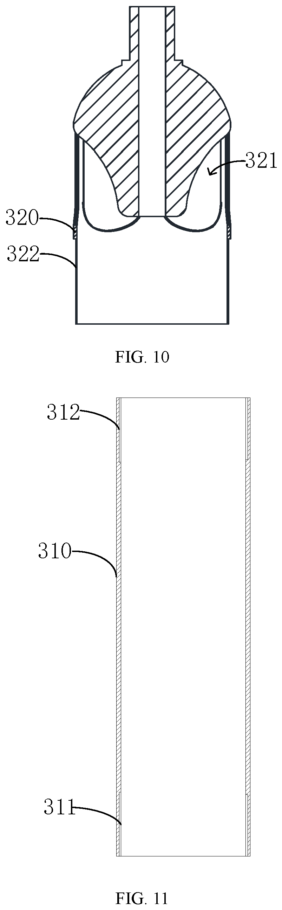

Referring to FIG. 5, FIG. 9 and FIG. 10, in an embodiment, the cannula assembly 300 further includes an outlet tube 320, the outlet tube 320 is fixedly connected to an end of the compensation tube 310 away from the housing 100, and the outlet tube 320 is provided with the second opening 321. A proximal end of the outlet tube 320 is provided with a third insertion portion 322, and a distal end of the compensation tube 310 is provided with a fourth insertion portion 312 (see FIG. 11). The third insertion portion 322 and the fourth insertion portion 312 are engaged with each other in an interference fit, so as to connect the compensation tube 310 and the outlet tube 320. An inner peripheral wall of the outlet tube 320 and an inner peripheral wall of the compensation tube 310 are aligned in an axial direction to facilitate smooth blood flow. An outer peripheral wall of the outlet tube 320 and an outer peripheral wall of the compensation tube 310 are aligned in an axial direction to provide the blood pump 10 with a smooth and regular overall profile, facilitating movement of the blood pump 10 within a blood vessel or the heart 20.

Referring to FIG. 5 and FIG. 6, in an embodiment, the blood pump 10 includes a catheter 12, and the catheter 12 is arranged through the proximal end of the housing 100 from the outside to the inside to be connected to the drive unit 200. The proximal end of the housing 100 may sleeve the catheter 12, the drive unit 200, or the connection between the catheter 12 and the drive unit 200, so as to be directly or indirectly connected to the drive unit 200.

Referring to FIG. 3, in an embodiment, the catheter 12 has a proximal portion 12a, a distal portion 12b, and a bending portion 12c. The bending portion 12c is located between the proximal portion 12a and the distal portion 12b and adjacent to the distal portion 12b, and the bending portion 12c is pre-formed into a bent shape, such that a shape of the bending portion 12c matches an anatomical structure of a first communication site of the right atrium 22. The first communication site refers to a communication site between the inferior vena cava 20 and the right atrium 22. Thus, the bending portion 12c of the catheter 12 can adapt to the shape of the communication site between the inferior vena cava 20 and the right atrium 22, allowing the blood pump 10 to enter the right atrium 22 from the inferior vena cava 20 via the first communication site, reducing the difficulty of the blood pump 10 passing through a curved opening of the first communication site, improving the smoothness of delivery of the blood pump 10, and reducing damage and discomfort caused by the blood pump 10 to the human body.

Certainly, in another embodiment, the shape of the bending portion 12c matches an anatomical structure of a second communication site of the right atrium 22. The second communication site refers to a communication site of the superior vena cava 21 and the right atrium 22. Thus, the shape of the bending portion 12c of the catheter 12 can be adapted to the shape of the communication site of the superior vena cava 21 and the right atrium 22, so that the blood pump 10 can enter the right atrium 22 from the superior vena cava 21 through the second communication site, difficulty of passing of the blood pump 10 through a bend of the second communication site is lowered, pushing smoothness of the blood pump 10 is improved, and damage and discomfort of the human body caused by the blood pump 10 are reduced.

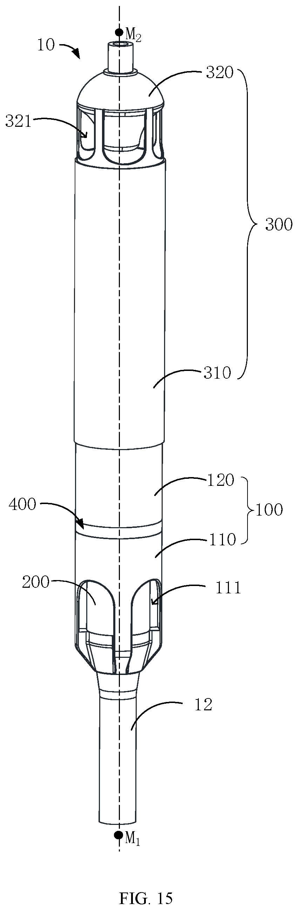

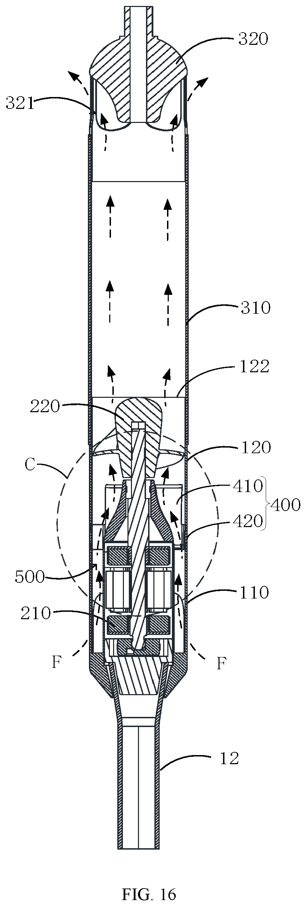

Referring to FIG. 15 and FIG. 16, in an embodiment, the blood pump 10 includes a housing 100 and a drive unit 200. The housing 100 is provided with a liquid inlet 111 and a liquid outlet 122. The drive unit 200 is arranged inside the housing 100, the drive unit 200 is spaced apart from an inner peripheral surface of the housing 100 to from a blood flow channel 500, and the blood flow channel 500 is in communication with the liquid inlet 111 and the liquid outlet 122. A proximal end of the housing 100 is fixedly connected to a proximal end of the drive unit 200 or to a catheter 12 of the blood pump 10, so as to maintain a stable relative position between the housing 100 and the motor 210, thereby forming the blood flow channel 500 with a stable structure. The drive unit 200 includes the motor 210 and an impeller 220 connected to a distal end of the motor 210.

Specifically, the blood flow channel 500 can be the flow passage gap 500 shown in FIG. 6; the liquid inlet 111 may be the first opening 111 of the housing 100 shown in FIG. 6; the liquid outlet 122 may be the distal tube port 122 of the housing 100 shown in FIG. 6. The motor 210 can drive the impeller 220 to rotate to drive blood to flow from the liquid inlet 111 to the liquid outlet 122 via the blood flow channel 500.

Since the blood flow channel 500 is formed between an outer periphery of the drive unit 200 and the inner peripheral surface of the housing 100, a part of the blood flow channel 500 between an outer peripheral surface of the motor 210 and the inner peripheral surface of the housing 100 is an annular passage. Thus, when the blood flows into the impeller 220 through the annular passage on an outer periphery of the motor 210, since a circulation area and a shape of the blood flow channel 500 are changed, a flow direction of the blood is changed to cause blood streams to collide and interfere with each other, i.e., a flow state of blood flow may be disordered, and kinetic energy of the blood may be lost.

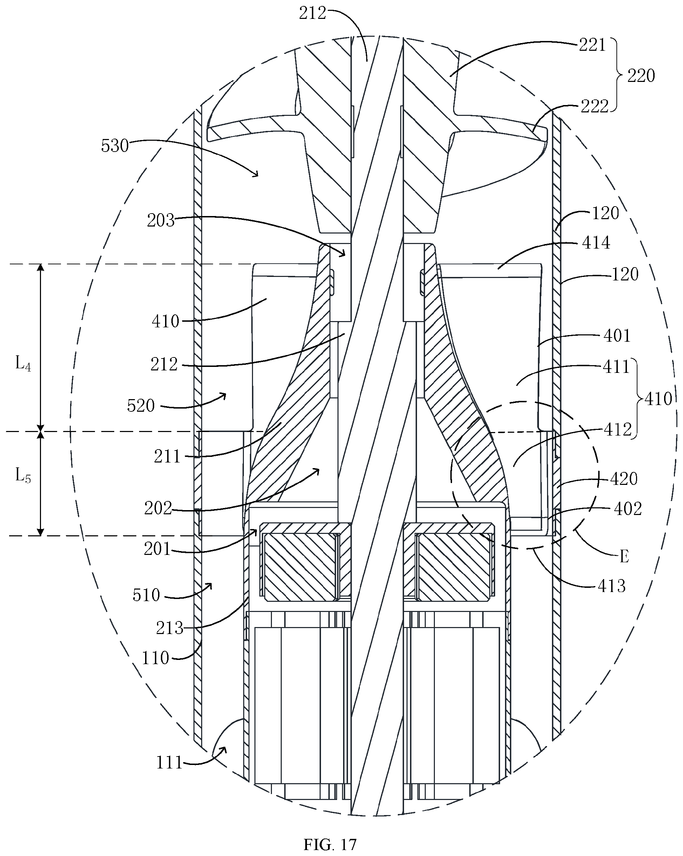

Referring to FIG. 16 and FIG. 17, in view of the above problem, in an embodiment, the blood pump 10 further includes a flow straightening device 400. The flow straightening device 400 is arranged in the blood flow channel 500, and the flow straightening device 400 can guide the blood to flow from the outer periphery of the motor 210 to the impeller 220. Specifically, the portion of the blood flow channel 500 located between the outer peripheral surface of the motor 210 and the inner peripheral surface of the housing 100 is an annular passage, which is relatively narrow. When the blood in the annular passage flows to the flow straightening device 400, the blood passes through the flow straightening device 400 and is straightened into an ordered fluid flowing in an axial direction, and is guided by the flow straightening device 400 to flow towards the impeller 220 in the axial direction, so that the blood is guided into the impeller 220 in an orderly manner, the loss of the kinetic energy when the blood enters the impeller 220 is reduced, the blood flow velocity driven by the impeller 220 is effectively increased, the blood flow rate driven by the impeller 220 is then increased, and the blood pumping efficiency of the blood pump 10 is improved.

It should be noted that although a direction of each of the blood streams is also changed in the impeller 200, the change in the direction when the impeller 220 moves the blood streams is regular and predictable. Compared with the fluid with disordered flow directions, resistance is obviously smaller when the fluid with stable flow directions is driven to move. The blood pump according to the present application is described in detail below in conjunction with the accompanying drawings and specific embodiments.

In an embodiment, the liquid outlet 122 of the blood pump 10 may be used directly as a blood outlet (as shown in FIG. 2). In this case, when the blood pump 10 is introduced into the right ventricle, the liquid inlet 111 of the blood pump 10 is located in the right ventricle 24, and the liquid outlet 122 of the blood pump 10 extends into the pulmonary artery 26. The blood pump 10 is capable of discharging blood from the liquid outlet 122 directly into the pulmonary artery 26. In this embodiment, the liquid outlet 122 may be formed in a side wall of a distal end of the housing 100.

It is considered here that the housing 100 and the drive unit 200 constitute a pump body 11 of the blood pump 10, and since the drive unit 200 is arranged inside the housing 100, an axial length of the pump body 11 is reduced. When the blood pump passes through the pulmonary valve 25 into the pulmonary artery 26, the pump body 11 of the blood pump 10 may be positioned on the pulmonary valve 25. However, the pulmonary valve 25 alternately opens and closes with pulsation of the heart, and as shown in FIG. 6, during systole of the heart 20, the pulmonary artery 26 opens, allowing blood to flow from the right ventricle 24 into the pulmonary artery 26, and in this case, a clamping force applied by the pulmonary valve 25 to the blood pump 10 is decreased, so that the pump body 11 of the blood pump 10 has a tendency to move towards the interior of the pulmonary artery 26 under an impact of the blood flowing in a direction Q₁. Similarly, as shown in FIG. 5, when the heart 20 dilates, the pulmonary valve 25 is closed, and the pulmonary valve 25 has a tendency to move the pump body 11 of the blood pump 10 towards the interior of the right ventricle 24 along a direction Q₂. Therefore, if the axial size of the pump body 11 of the blood pump 10 is excessively short, the pump body 11 of the blood pump 10 is prone to fall off from the pulmonary valve 24 during the alternating process of opening and closing of the pulmonary valve 24, which affects use safety and reliability of the blood pump 10.

With reference to FIG. 15 to FIG. 17, in view of the above, in another embodiment, the blood pump 10 may further include a cannula assembly 300, and the cannula assembly 300 is provided with a liquid discharge port 321. The cannula assembly 300 includes a compensation tube 310, and the compensation tube 310 is fixedly connected to and in communication with the distal end of the housing 100, such that the liquid discharge port 321 is in communication with the liquid outlet 122 of the housing 100 through an inner cavity of the compensation tube 310.

As shown in FIG. 16, in this case, the liquid discharge port 321 serves as the blood outlet. The liquid discharge port 321 may be the second opening 321 of the housing 100 shown in FIG. 6. When the blood pump 10 is introduced into the right ventricle, the liquid inlet 111 of the blood pump 10 is located in the right ventricle, and the liquid discharge port 321 of the blood pump 10 is located in the pulmonary artery; after the blood pump 10 is started, the blood in the right ventricle 24 enters the blood flow channel 500 of the blood pump 10 from the liquid inlet 111, is discharged from the blood flow channel 500 to the inner cavity of the compensation tube 310 through the liquid outlet 122, then flows to the liquid discharge port 321 through the inner cavity of the compensation tube 310, and finally is discharged to the pulmonary artery 26 from the liquid discharge port 321.

Therefore, after the compensation tube 310 of the blood pump 10 according to the present application enters the right ventricle 24 before the pump body 11, the pump body 11 with a short length can enter the right ventricle 24 with the compensation tube 310 only through small elastic deformation of the compensation tube 310. Due to the short axial length of the pump body 11 and the elastic deformation of the compensation tube 310, the pump body 11 of the blood pump 10 can deflect upwards in the right ventricle and then pass through the pulmonary valve 25 into the pulmonary artery 26, so that the liquid inlet 111 can be located in the right ventricle 24, and the liquid discharge port 321 of the blood pump 10 can be located in the pulmonary artery 26. Moreover, since the compensation tube 310 and the pump body 11 are axially arranged, not only the pump body 11 can be clamped by the pulmonary valve 25, but also the compensation tube 310 can be clamped by the pulmonary valve 25, so that the axial length of the blood pump 10 for clamping and positioning by the pulmonary valve can be effectively increased. Therefore, after the blood pump 10 passes through the pulmonary valve 25, the pulmonary valve 25 can clamp at least one of the pump body 11 or the compensation tube 310 during the process that the pulmonary valve 25 alternately opens and closes with the pulsation of the heart, so that the risk of the blood pump 10 becoming dislodged from the pulmonary valve 25 can be reduced.

Obviously, in the present application, the cannula assembly 300 is not necessary, as long as the axial length of the housing 100 is large enough and the pump body 11 is not prone to fall off from the pulmonary valve 25.

Referring to FIG. 16 and FIG. 17, in an embodiment, the motor 210 includes a motor body 213 and a shaft support base 211 connected to a distal end of the motor body 213. The motor 210 further includes a motor shaft 212, and the motor shaft 212 extends out from the motor body 213 and passes through the shaft support base 211 to be connected to the impeller 220. Optionally, the blood flow channel 500 includes a flow guide section 510, a flow straightening section 520, and a pressurization section 530 that are sequentially in communication. The flow guide section 510 is located between an outer peripheral surface of the motor body 213 and the inner peripheral surface of the housing 100, and is in communication with the liquid inlet 111. The flow straightening section 520 is located between an outer peripheral surface of the shaft support base 211 and the inner peripheral surface of the housing 100. The pressurization section 530 is located between the impeller 220 and the inner peripheral surface of the housing 100, and is in communication with the liquid outlet 122. The flow straightening device 400 is arranged in the flow straightening section 520, so that blood delivered from the flow guide section 510 is straightened into an ordered fluid flowing in the axial direction, and is then guided into the pressurization section 530 to be driven by the impeller 200.

Specifically, the motor 210 has the motor shaft 212, and the motor shaft 212 extends out from the distal end of the motor 210 and passes through the shaft support base 211 to be connected to the impeller 220. A proximal end of the shaft support base 211 is connected to the distal end of the motor 210 and covers the distal end of the motor 210. The flow straightening device 400 is fixedly connected to the outer peripheral surface of the shaft support base 211.

Since the flow guide section 510 is formed by causing the motor 210 to be spaced apart from the housing 100, and the motor 210 has a substantially regular shape, the flow guide section 510 is a relatively regular annular passage, and a flow area and a shape of each part of the flow guide section 510 are relatively regular. After blood F flows out from a distal end of the flow guide section 510, the blood F is prone to be disturbed at the distal end of the flow guide section 510 due to changes of a flow area and a flow channel between the flow guide section 510 and the pressurization section 530 and a suction force of the impeller 220. Therefore, in this embodiment, the flow straightening section 520 is arranged between the flow guide section 510 and the pressurization section 530 for transition, and the flow straightening device 400 is arranged in the flow straightening section 520, so that when the blood pump 10 works, the blood F firstly enters the flow guide section 510 of the blood flow channel 500 from the liquid inlet 111, then sequentially flows through the flow straightening section 520 and the pressurization section 530 of the blood flow channel 500 and flows to the liquid outlet 122. Within the flow straightening section 520, the flow straightening device 400 can straighten the blood flowing out from the distal end of the flow guide section 510, thereby straightening the blood into an ordered fluid flowing in the axial direction, and guiding the fluid in an orderly manner into the impeller 220 of the pressurization section 530.

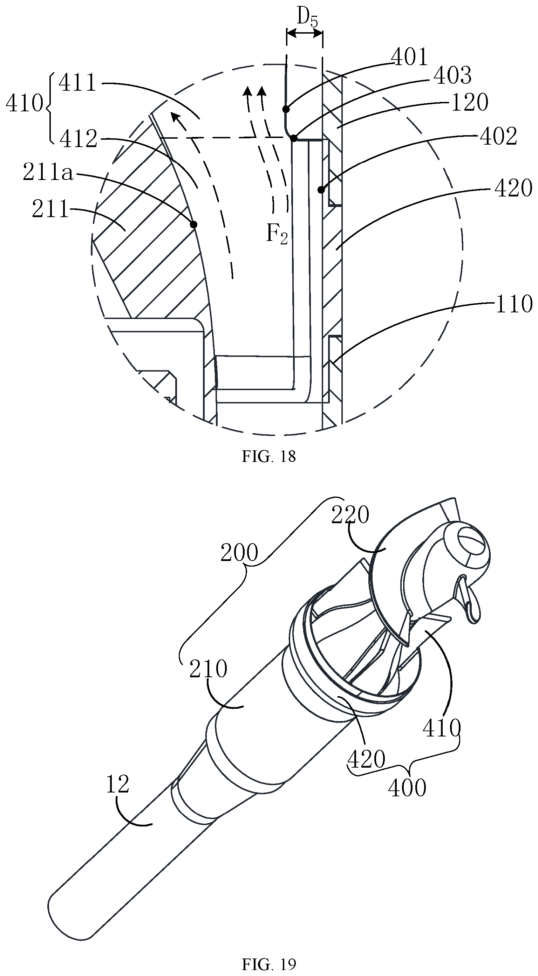

Referring to FIG. 16 to FIG. 18, in an embodiment, the impeller 220 includes a hub 221 and blades 222 arranged on an outer periphery of the hub 221. An outer diameter of the motor body 213 is greater than that of the hub 221. Preferably, an outer diameter of the shaft support base 211 gradually decreases in a direction from the motor 210 to the impeller 220, so that the outer peripheral surface of the shaft support base 211 forms a tapered flow guide surface 211a. The tapered flow guide surface 211a can guide blood in a flow straightening channel 404 to gradually deflect towards a central axis of the impeller 220, and then flow to a central region of the impeller 220, i.e., the shaft support base 211 can guide the blood to gradually flow toward the central region of the impeller 220, so as to accommodate the radial size difference between the motor 210 and the hub 221 of the impeller 220.

Referring to FIG. 16 to FIG. 18, further, the flow straightening device 400 includes a plurality of flow straightening vanes 410. The plurality of flow straightening vanes 410 are spaced apart on the tapered flow guide surface 211a in a circumferential direction of the shaft support base 211. The flow straightening vanes 410 may be integrally formed with the shaft support base 211. Specifically, the plurality of flow straightening vanes 410 are fixed on the tapered flow guide surface 211a at intervals in the circumferential direction of the shaft support base 211, and each two adjacent flow straightening vanes 410 are spaced apart to form a flow straightening channel 404. When the blood flows into the flow straightening device 400, the blood is partitioned by the flow straightening vanes 410 and flows through the flow straightening channels 404 on both sides of the flow straightening vanes 410, and the blood passing through the flow straightening channels 404 can also be guided by the outer peripheral surface of the shaft support base 211 to flow towards the impeller 220, which facilitates accelerating the blood flow through the flow straightening channels 404.

Optionally, an outer side edge of the flow straightening vane 410 away from the tapered flow guide surface 211a is parallel to the motor shaft 212. Such a configuration, the blood in each flow straightening channel 404 can be limited between the two corresponding flow straightening vanes 410 and the tapered flow guide surface 211a, and the two flow straightening vanes 410 and the tapered flow guide surface 211a can jointly guide the blood in the flow straightening channel 404 to flow towards the impeller 220, which is beneficial to accelerating flowing of the blood into the impeller 220 and increasing the flow velocity of the blood. Since the outer side edge of the flow straightening vane 410 away from the tapered flow guide surface 211a is parallel to the motor shaft 212, the flow straightening vane 410 can have a large flow guide area, and can guide more blood to flow. The outer side edge may be formed by a first side edge 401 and a second side edge 402. It should be noted that, in other embodiments, the flow straightening vane 410 of the flow straightening device 400 may not be fixedly connected to the shaft support base 211, but is fixedly connected to the inner peripheral surface of the housing 100.

Referring to FIG. 16 to FIG. 18, in an embodiment, the flow straightening device 400 includes a plurality of flow straightening vanes 410, and the plurality of flow straightening vanes 410 are fixed on the outer peripheral surface of the motor 210 and spaced apart in a circumferential direction of the motor 210. The plurality of flow straightening vanes 410 each extend in the axial direction of the motor 210 to form a linearly extending flow straightening channel 404 between each two adjacent flow straightening vanes 410, i.e., the flow straightening channels 404 extend linearly in the axial direction of the motor 210 to guide the blood to flow into the impeller 220 in the axial direction. In this way, each two adjacent flow straightening vanes 410 may define one flow straightening channel 404, so that the flow straightening device 400 has a plurality of linearly extending flow straightening channels 404 which are spaced apart in the circumferential direction of the shaft support base 211. As described above, the flow straightening vanes 410 may be fixedly connected to the outer peripheral surface of the shaft support base 211 or the inner peripheral surface of the housing 100.

Based on this, when the blood flows to the flow straightening device 400, the blood is divided into a plurality of ordered blood streams axially flowing stably by the plurality of flow straightening channels 404 of the flow straightening device 40, and the ordered blood streams axially flow into the impeller 220. Preferably, the plurality of flow straightening channels 404 correspond to spacing regions between a plurality of blades 222 of the impeller 220 respectively, so that each flow straightening channel 404 directs the blood into the corresponding spacing region to reduce collision of the blood with end portions of the blades 222.

Referring to FIG. 17 to FIG. 20, in an embodiment, the flow straightening vane 410 includes a distal vane body 411 and a proximal vane body 412. The distal vane body 411 and the proximal vane body 412 are arranged in the axial direction of the motor 210 and connected to each other. The distal vane body 411 is adjacent to the impeller 220. The distal vane body 411 has a first side edge 401 away from the outer peripheral surface of the motor 210, and the proximal vane body 412 has a second side edge 402 away from the outer peripheral surface of the motor 210. The second side edge 402 and the first side edge 401 extend in the same direction, and the first side edge 401 and the second side edge 402 jointly form an outer side edge of the flow straightening vane 410.

Optionally, at least one of the first side edge 401 or the second side edge 402 is fixedly connected to the inner peripheral surface of the housing 100. Such a configuration, the flow straightening vane 410 can guide the blood to flow towards the impeller 220 in the axial direction and be supported between the drive unit 200 and the housing 100 to ensure that the stable blood flow channel 500 is formed between the drive unit 200 and the housing 100, so as to facilitate the blood flow channel 500 to stably convey the blood.

Optionally, the flow straightening vane 410 may be fixedly connected to the inner peripheral surface of the housing 100 through the first side edge 401; alternatively, the flow straightening vane 410 may be fixedly connected to the inner peripheral surface of the housing 100 through the second side edge 402; alternatively, the first side edge 401 and the second side edge 402 are both fixedly connected to the inner peripheral surface of the housing 100.

Referring to FIG. 16 and FIG. 17, since the distal vane body 411 is adjacent to the impeller 220, after the blood enters from an inlet end of the flow straightening channel 404, a part of the blood flows to the distal vane body 411 along the proximal vane body 412 of the flow straightening vane 410 in an attached manner, and then is guided by the distal vane body 411 to flow into the impeller 220. It is considered here that, if the first side edge 401 of the distal vane body 411 is also fixedly connected to the inner peripheral surface of the housing 100, an entire surface of the flow straightening vane 410 is joined to the inner peripheral surface of the housing 100, and the blood flowing towards the distal vane body 411 along the proximal vane body 412 in the attached manner may adhere to the inner peripheral surface of the housing 100, and is thus attached to the inner peripheral surface of the housing 100, not easily introduced onto the impeller 220 and not easily driven by the impeller 220.

Referring to FIG. 17, FIG. 18 and FIG. 20, in view of the above, in this embodiment, the first side edge 401 is spaced apart from the inner peripheral surface of the housing 100, and the second side edge 402 is fixedly connected to the inner peripheral surface of the housing 100. Specifically, the second side edge 402 of the proximal vane body 412 protrudes radially relative to the first side edge 401 of the distal vane body 411, so that the second side edge 402 can be connected to the inner peripheral surface of the housing 100 to support the housing 100. The first side edge 401 is spaced apart from the inner peripheral surface of the housing 100 by a short distance D₅, so that a little of the blood F₂ flowing along the proximal vane body 412 in the attached manner adheres to the inner peripheral surface of the housing 100 during transition to the distal vane body 411, more blood F₂ can be guided into the impeller 220 by the distal vane body 411, and then, the flow straightening channel 404 guides most of the blood into the impeller 220 for driving by the impeller 220, thereby increasing the flow rate of the blood driven by the impeller 220 and improving the blood pumping efficiency.

Further, the first side edge 401 extends linearly in the axial direction, so that the flow straightening vane 410 has a large flow straightening surface. Further, an axial length of the first side edge 401 is greater than that of the second side edge 402. Specifically, the first side edge 401 has a fourth axial length L₄, and the second side edge 402 has a fifth axial length L₅, i.e., L₄>L₅. Such a configuration, an extension length of the distal vane body 411 may be greater than that of the proximal vane body 412, so that the blood guided by the proximal vane body 412 is in less contact with the inner peripheral surface of the housing 100, and more blood is guided to the distal vane body 411, which is beneficial to increasing an amount of the blood guided by the distal vane body 411 to the impeller 220.

Since the second side edge 402 of the proximal vane body 412 protrudes radially relative to the first side edge 401 of the distal vane body 411, a transition step may be formed at the connection between the second side edge 402 and the first side edge 401. Therefore, optionally, a rounded corner 403 may be arranged at the connection between the second side edge 402 and the first side edge 401 to avoid a narrow sharp corner at the connection, which not only prevents blood cell damage due to blood impingement but also facilitates the proximal vane body 412 in guiding the blood to the distal vane body 411.

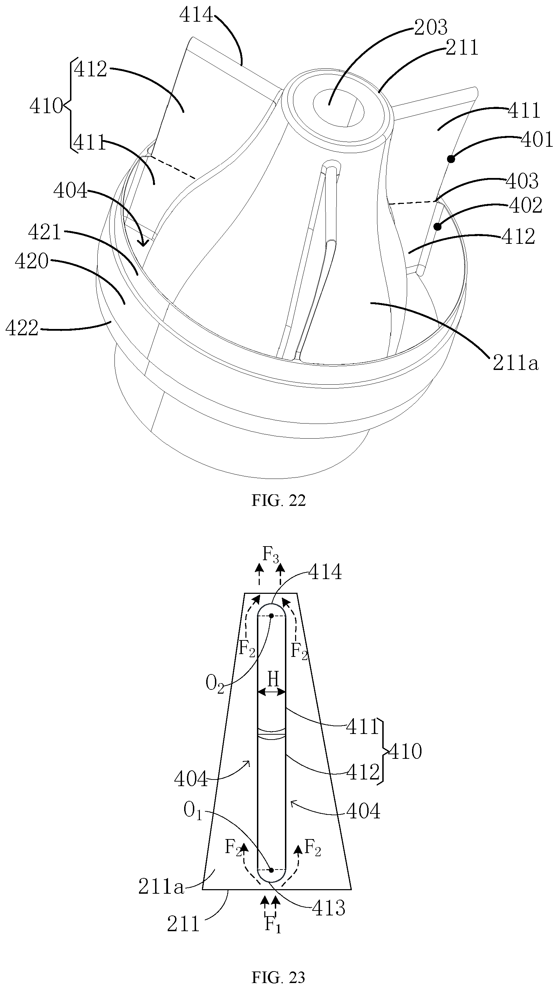

Referring to FIG. 17, FIG. 22 and FIG. 23, in an embodiment, the flow straightening vane 410 has an inflow end face 413 and an outflow end face 414. The inflow end face 413 and the outflow end face 414 are respectively located at two ends of the flow straightening vane 410, the outflow end face 414 faces toward the impeller 220, and the inflow end face 413 faces away from the impeller 220. When the blood enters the flow straightening device 400, a portion of the blood F₁ initially contacts the inflow end face 413 of the flow straightening vane 410, and then is divided into two blood streams F₂ by the inflow end face 413, and the two blood streams flow to the flow straightening channels 404 on the two sides of the flow straightening vane 410 respectively. Subsequently, the blood streams F₂ flow along the flow straightening channels 404, and when reaching the outlets of the flow straightening channels 404, the blood streams F₂ from the two adjacent flow straightening channels 404 merge into a blood stream F₃ at the outflow end face 414 of the flow straightening vane 410.

In view of this, optionally, at least one of the inflow end face 413 or the outflow end face 414 is configured in an arc shape from one vane surface to the other vane surface of the flow straightening vane 410. Specifically, profiles of the inflow end face 413 and the outflow end face 414 along a thickness direction of the flow straightening vane 410 are semicircular, and a center O₁ of an arc where the inflow end face 413 is located is located on a bisector of a thickness of the flow straightening vane 410, so that two sides of the inflow end face 413 are smoothly joined to the two vane surfaces of the flow straightening vane 410 respectively. Similarly, a center O₂ of an arc where the outflow end face 414 is located is also located on the bisector of the thickness of the flow straightening vane 410, and both sides of the outflow end face 414 are also smoothly joined to the two vane surfaces of the flow straightening vane 410. Such a configuration, the inflow end face 413 or the outflow end face 414 can be smoother, and then, resistance of the inflow end face 413 or the outflow end face 414 to the fluid can be reduced.

Taking the inflow end face 413 as an example, when the blood enters the flow straightening device 400, the blood F₁ contacting the inflow end face 413 of the flow straightening vane 410 first is divided into the two blood streams F₂ along an arc direction of the inflow end face 413 under guidance of the arc-shaped inflow end face 413, and the two blood streams F₂ respectively flow into the flow straightening channels 404 on both sides of the flow straightening vane 410, so that blockage of the blood by the inflow end face 413 is effectively reduced, the loss of the kinetic energy when the blood enters the flow straightening device 400 is reduced, and the blood pumping efficiency is improved. Similarly, when the blood streams F₂ of the two adjacent flow straightening channels 404 are discharged, under guidance of the arc-shaped outflow end face 414, the two blood streams F₂ gradually fill and converge to a distal space of the flow straightening vane 410 along an arc direction of the outflow end face 414, so as to converge into the blood flow F₃, which relatively improves smoothness of a flow condition after the blood leaves the flow straightening vane 410.

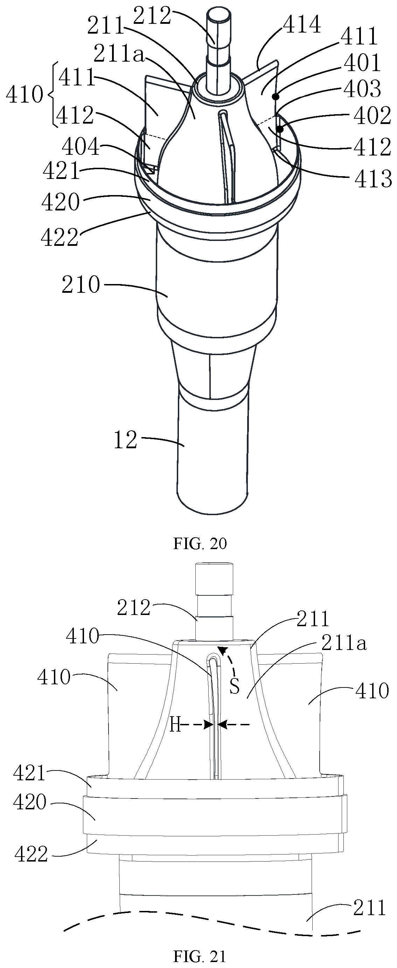

Referring to FIG. 21 to FIG. 23, in an embodiment, the thickness H of the flow straightening vane 410 gradually increases in the direction from the motor 210 to the impeller 220, and thus, a space in the flow straightening section 520 between two flow straightening vanes 410 gradually decreases in the direction from the motor 210 to the impeller 220, so as to form the flow straightening channel 404 for pressurizing the blood, i.e., a width of the flow straightening channel 404 gradually decreases along the direction from the motor 210 to the impeller 220, so that the flow straightening channel 404 can pre-pressurize the blood stream F₂ and then guide the pre-pressurized blood stream to the impeller 220, i.e., the blood can be pre-pressurized in the flow straightening section 520 through the flow straightening channel 404, and then, the blood can be further pressurized by the impeller 220 after flowing into the pressurization section 530. In this way, compared with pressurization only by the impeller 220, gradual pressurization of the blood in the flow straightening section 520 and the pressurization section 530 facilitates the blood to be pressurized to a higher pressure, and a blood discharge efficiency can be improved, i.e., this arrangement improves the blood pumping efficiency of the blood pump 10.

Referring to FIG. 21, in another embodiment, the distal portions 12b of the plurality of flow straightening vanes 410 are all curved towards a same side in the circumferential direction of the motor 210, and a curving direction is the same as a rotation direction of the impeller 220. Thus, the blood can be gradually subjected to an initial movement force with a direction consistent with the rotation direction of the impeller 220 in the process of flowing from the proximal end to the distal end of the flow straightening vane 410. This arrangement can improve convenience of driving of movement of the blood by the impeller 220, i.e., reduce resistance generated when the impeller 220 drives the blood to flow, so as to improve the blood pumping efficiency of the blood pump 10. The distal end of the flow straightening vane 410 is bent in the direction S shown in FIG. 21.

Referring to FIG. 16 to FIG. 18, in an embodiment, the flow straightening device 400 further includes a fixing ring 420. The fixing ring 420 surrounds an outer periphery of the flow straightening vane 410, the fixing ring 420 is fixedly connected to the housing 100, and at least a part of an inner wall surface of the fixing ring 420 is fixedly connected to the flow straightening vane 410 to support the housing 100. The fixing ring 420 in FIG. 16 to FIG. 18 has a same structure as the fixing ring 420 shown in FIG. 6, i.e., the flow straightening vane 410 in this embodiment may be used as the support member 410 shown in FIG. 6 to support the fixing ring 420.

Specifically, the fixing ring 420 surrounds an outer periphery of the proximal vane body 412 of the flow straightening vane 410; the inner wall surface of the fixing ring 420 is spaced apart from the outer peripheral surface of the shaft support base 211, and at least a part of the inner wall surface of the fixing ring 420 is fixedly connected to the proximal vane body 412 of the flow straightening vane 410, so that the motor 210 supports and fixes the fixing ring 420 through the flow straightening vane 410, and the housing 100 is supported by the fixing ring 420, thereby constructing the stable blood flow channel 500 between the housing 100 and the drive unit 200.

Referring to FIG. 16, FIG. 17 and FIG. 20, in an embodiment, the housing 100 includes an inlet tube 110 and a connection tube 120. The inlet tube 110 and the connection tube 120 are connected to two ends of the fixing ring 420 respectively, and coaxially arranged with the fixing ring 420, so that the fixing ring 420 constitutes a middle portion of the housing 100. "Coaxial" means that central axes of the inlet tube 110, the fixing ring 420 and the connection tube 120 are located on a same straight line. The inlet tube 110, the fixing ring 420 and the connection tube 120 are in butt connection in sequence along the direction from the motor 210 to the impeller 220, thereby forming the housing 100 by splicing, so that the fixing ring 420 constitutes the middle portion of the housing 100. The inlet tube 110 is provided with the liquid inlet 111; the connection tube 120 is provided with the liquid outlet 122.

During assembly, the inlet tube 110 of the housing 100 can be fitted over the outer periphery of the proximal end of the motor 210 from the proximal end of the motor 210 and fixedly connected to the proximal end of the fixing ring 420; and then, the connection tube 120 of the housing 100 is fitted over the outer periphery of the distal end of the motor 210 from the distal end of the motor 210 and fixedly connected to the distal end of the fixing ring 420, so that the housing 100 and the motor 210 are fixedly connected, thus improving the assembly convenience.

Referring to FIG. 16 and FIG. 17, in an embodiment, the inlet tube 110 includes a straight tube 112, a diameter-variable tube 113, and a plurality of connection arms 114 connecting the straight tube 112 and the diameter-variable tube 113, as shown in the embodiment shown in FIG. 6 to FIG. 9. The straight tube 112 is connected to the fixing ring 420. The diameter-variable tube 113 is sleeved over the outer periphery of the connection between the catheter 12 and the drive unit 200, and is fixedly connected to at least one of the drive unit 200 or the catheter 12. The plurality of connection arms 114 are spaced apart in a circumferential direction of the straight tube 112, and one liquid inlet 111 is formed between each two adjacent connection arms 114.

Specifically, the straight tube 112 is configured as a straight tube shape, so that an inner peripheral surface of the straight tube 112 forms a cylindrical surface, reducing fluid resistance in the blood flow channel. The diameter-variable tube 113 is configured in a conical shape. By sleeving the diameter-variable tube 113 over the outer periphery of the connection between the catheter 12 and the drive unit 200, the connection can be encapsulated within the diameter-variable tube 113, preventing adhesive at the connection from being washed away by blood. The diameter-variable tube 113 has two ports, one of which is in interference fit with the outer peripheral surface of the distal end of the catheter 12, and the other of which is in interference fit with the outer peripheral surface of the proximal end of the drive unit 200. The straight tube 112, the diameter-variable tube 113, and the connection arms 114 may be integrally formed.

Further, inner wall surfaces of the straight tube 112, the fixing ring 420 and the connection tube 120 are aligned in the axial direction to facilitate smooth blood flow. Moreover, outer wall surfaces of the straight tube 112, the fixing ring 420 and the connection tube 120 are also aligned in the axial direction. Such a configuration, the straight tube 112, the fixing ring 420 and the connection tube 120 together form a regular cylindrical body. Such a configuration, in one aspect, a smooth and regular outer peripheral surface of the housing 100 is provided, thus reducing damage to human tissue during the implantation process of the blood pump 10. In the other aspect, a smooth and regular inner peripheral surface of the housing 100 is also provided, thus reducing the fluid resistance in the blood flow channel 500, minimizing the loss of the kinetic energy of the blood flowing in the blood flow channel 500, improving the blood pumping efficiency, and reducing damage to blood cells.

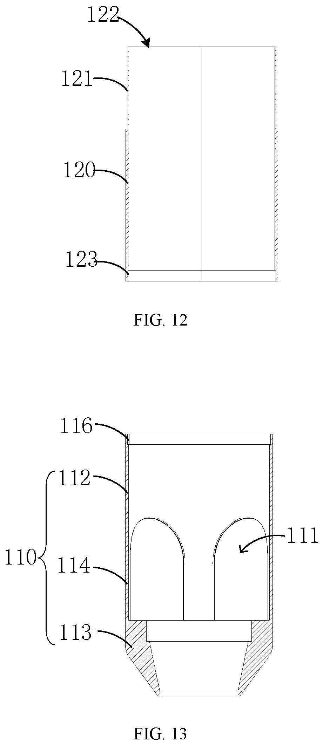

Referring to FIG. 16 and FIG. 17, in an embodiment, the connection tube 120 is provided with a first insertion portion 121 (see FIG. 12), a compensation tube 310 is provided with a second insertion portion 311 (see FIG. 11), and the first insertion portion 121 and the second insertion portion 311 are in insertion fit to communicate the blood flow channel 500 in the housing 100 with an inner cavity of the compensation tube 310.

The fixing ring 420 is the same as the fixing ring 420 shown in FIG. 11, the fixing ring 420 is also provided with a seventh insertion portion 421 and an eighth insertion portion 422, and the seventh insertion portion 421 is engaged with a fifth insertion portion 123 of the connection tube 120 in an interference fit; the eighth insertion portion 422 is engaged with a sixth insertion portion 116 of the inlet tube 110 in an interference fit. Such a configuration, the fixing ring 420 is inserted into the connection tube 120 and the inlet tube 110 respectively, so as to form the housing 100.

As shown in FIG. 16, the compensation tube 310 shown in FIG. 16 may be the same as the compensation tube shown in FIG. 6 and also has a first axial length L₁, and the first axial length L₁ is greater than or equal to 20mm and less than or equal to 35mm, i.e., 20mm≤L₁≤35mm. The structure of the compensation tube 310 and the connection and fitting between the compensation tube 310 and the housing 100 in this embodiment can be implemented by referring to the implementation of the compensation tube 310 in the embodiment shown in FIG. 5 to FIG. 14, and are not repeated herein.

Referring to FIG. 15 and FIG. 16, the cannula assembly 300 further includes an outlet tube 320, and a liquid discharge port 321 is formed in the outlet tube 320. A distal end of the compensation tube 310 is provided with a fourth insertion portion 312, a proximal end of the outlet tube 320 is provided with a third insertion portion 322, and the fourth insertion portion 312 is engaged with the third insertion portion 322 in an interference fit, so that the inner cavity of the compensation tube 310 is in communication with an inner cavity of the outlet tube 320, and the blood is conveyed to the outlet tube 320 and discharged to the pulmonary artery from the liquid discharge port 321.

Referring to FIG. 16 and FIG. 17, in an embodiment, a flushing flow channel 201 is provided inside the motor body 213. The shaft support base 211 is provided with a drainage cavity 202 and a shaft hole 203 in communication with the drainage cavity 202, and the drainage cavity 202 is in communication with the flushing flow channel 201. The motor shaft 212 extends out from the distal end of the motor body 213, and passes through the drainage cavity 202 and the shaft hole 203 of the shaft support base 211 sequentially to be connected to the impeller 220. It is easily understood that the flow path of the flushing fluid can be substantially the same as an extension direction of the motor shaft 212, i.e., the flushing fluid can flow into the drainage cavity 202 through the flushing flow channel 201 and then flow out from the shaft hole 203 of the drainage cavity 202 into a gap between the shaft support base 211 and the impeller 220, so that the flushing fluid can wash away blood between the end of the shaft support base 211 and the end of the impeller 220, reducing the risk of blood remaining in the gap between the shaft support base 211 and the impeller 220 and forming thrombus.

Further, a diameter of the drainage cavity 202 gradually decreases in the direction from the motor 210 to the impeller 220, so that the drainage cavity 202 can guide the flushing fluid to the shaft hole 203 through the flushing flow channel 201, the flushing fluid is gradually pressed to increase a fluid pressure in the process that the flushing fluid flows from a proximal end to a distal end in the drainage cavity 202, and therefore, the flushing fluid can be discharged outside through the shaft hole 203, but the blood in the blood flow channel 500 cannot enter the drainage cavity 202 through the shaft hole 203, thus avoiding thrombus formation in the shaft hole 203 and improving a thrombus prevention effect.

In an embodiment, the blood pump further includes a catheter 12, a flushing fluid pipe (not shown, the same applies below) for circulation of the flushing fluid is provided in the catheter 12, and the flushing fluid pipe is in communication with the flushing flow channel 201 in the motor 210 to continuously supply the flushing fluid into the flushing flow channel 201, thereby continuously irrigating the gap between the shaft support base 211 and the impeller 220.

Referring to FIG. 6 and FIG. 15, in an embodiment, a distal end of the catheter 12 is connected to the drive unit 200. The proximal end of the housing 100 can be sleeved over the outer periphery of the connection between the catheter 12 and the drive unit 200, so as to encapsulate the connection, reducing flushing of the connection by the blood and preventing loosening of the adhesive at the connection. Referring to FIG. 3, the catheter 12 can also have a proximal portion 12a, a distal portion 12b, and a bending portion 12c. The bending portion 12c is located between the proximal portion 12a and the distal portion 12b and adjacent to the distal portion 12b, and the bending portion 12c is pre-formed into a bent shape, such that a shape of the bending portion 12c matches an anatomical structure of a first communication site or a second communication site of the right atrium 22. For details, reference may be made to the implementation manners described in the foregoing description and the embodiment shown in FIG. 3, and details are not described herein again.

The technical features in the above embodiments may be combined arbitrarily. For concise description, not all possible combinations of the technical features in the above embodiments are described. However, provided that they do not conflict with each other, all combinations of the technical features are to be considered to be within the scope of protection of the present application.

The above-mentioned embodiments only describe several implementations of the present application, and their description is specific and detailed, but should not be understood as a limitation on the protection scope of the present application. It should be noted that, for a person of ordinary skill in the art, various variations and improvements can be further made without departing from the conception of the present application, and these all fall within the protection scope of the present application. Therefore, the protection scope of the present application should be subject to the appended claims.

## Claims

1. A blood pump, comprising:
a housing;
a drive unit arranged inside the housing, a proximal end of the housing being fixedly connected to a proximal end of the drive unit or a catheter of the blood pump, and an inner peripheral surface of the housing being spaced apart from the drive unit to form a flow passage gap; and
a cannula assembly, the cannula assembly comprising a compensation tube, the compensation tube being elastic, the compensation tube being fixedly connected to a distal end of the housing, and an inner cavity of the compensation tube being in communication with the flow passage gap to form a blood flow channel;
wherein the proximal end of the housing is provided with a first opening, a distal end of the cannula assembly is provided with a second opening, and the second opening is in communication with the first opening via the blood flow channel.

2. The blood pump according to claim 1, wherein the blood pump further has at least one of the following features:
the compensation tube has a first axial length, and the first axial length is greater than or equal to 20mm and less than or equal to 35mm;
the compensation tube has a first axial length, the housing has a second axial length, and a ratio of the first axial length to the second axial length is greater than or equal to 0.7 and less than or equal to 1.3; or
the compensation tube comprises an inner membrane, an outer membrane, and an elastic coil arranged between the inner membrane and the outer membrane.

3. The blood pump according to claim 1, wherein the compensation tube is configured as a straight tube extending in an axial direction of the drive unit, and central axes of the compensation tube, the housing and the drive unit are located on a same straight line.

4. The blood pump according to claim 1, wherein the housing has a distal tube port in communication with the compensation tube; the drive unit comprises a motor and an impeller connected to the motor, the impeller has a distalmost point farthest from the catheter, and the distalmost point is located within the housing and does not extend beyond the distal tube port, such that the impeller does not extend into the compensation tube; and the distalmost point and an end face of the distal tube port are both located on a first plane, the first plane being a plane perpendicular to an axis of the drive unit.

5. The blood pump according to claim 1, wherein the distal end of the housing is provided with a first insertion portion, a proximal end of the compensation tube is provided with a second insertion portion, the second insertion portion is sleeved over an outer peripheral surface of the first insertion portion, and an overlapping region of the second insertion portion and the first insertion portion has a third axial length; the compensation tube has a first axial length, and a ratio of the third axial length to the first axial length is greater than or equal to 1/2 and less than or equal to 2/3.

6. The blood pump according to claim 1, wherein the drive unit comprises a motor and an impeller connected to the motor, a maximum outer diameter of the motor is a first diameter, an outer diameter of the impeller is a second diameter, and an inner diameter of the housing is a third diameter, the third diameter being greater than the second diameter, and the second diameter being greater than the first diameter.

7. The blood pump according to claim 1, wherein the housing comprises an inlet tube, a fixing ring, and a connection tube, the inlet tube and the connection tube being connected to two ends of the fixing ring respectively, the inlet tube being provided with the first opening, an end of the inlet tube away from the fixing ring being fixedly connected to the catheter or the proximal end of the drive unit, an end of the connection tube away from the fixing ring being connected to the compensation tube, and an inner wall surface of the fixing ring being fixedly connected to an outer peripheral surface of a motor of the drive unit through a support member.

8. The blood pump according to claim 7, wherein the inlet tube comprises a straight tube, a diameter-variable tube, and a plurality of connection arms connecting the straight tube and the diameter-variable tube, the straight tube being connected to the fixing ring, the diameter-variable tube being sleeved over an outer periphery of a connection between the catheter and the drive unit, and fixedly connected to at least one of the drive unit or the catheter, the plurality of connection arms being spaced apart in a circumferential direction of the straight tube, and one first opening being formed between each two adjacent connection arms.

9. The blood pump according to claim 8, wherein inner wall surfaces of the straight tube, the fixing ring and the connection tube are aligned in an axial direction of the straight tube, and outer wall surfaces of the straight tube, the fixing ring and the connection tube are also aligned in the axial direction of the straight tube; and
wherein each connection arm comprises a first connection portion connected to the straight tube, and a second connection portion connecting the first connection portion and the diameter-variable tube, the second connection portion being located on an outer peripheral surface of the diameter-variable tube, a notch groove being formed between each two adjacent second connection portions, and the notch groove being in communication with the first opening in an axial direction of the inlet tube.

10. The blood pump according to claim 1, wherein a distal end of the blood pump is capable of passing through the right atrium, the right ventricle and the pulmonary valve sequentially to extend into the pulmonary artery, such that the first opening is located in the right ventricle, and the second opening is located in the pulmonary artery; and
the blood pump comprises the catheter, the catheter having a proximal portion, a distal portion and a bending portion, the bending portion being located between the proximal portion and the distal portion and adjacent to the distal portion, and the bending portion being pre-formed into a bent shape, such that a shape of the bending portion matches an anatomical structure of a first communication site or a second communication site of the right atrium, wherein the first communication site is a communication site between the right atrium and the inferior vena cava, and the second communication site is a communication site between the right atrium and the superior vena cava.

11. A blood pump, comprising:
a housing, the housing being provided with a liquid inlet and a liquid outlet;
a drive unit arranged inside the housing, a proximal end of the housing being fixedly connected to a proximal end of the drive unit or a catheter of the blood pump, an inner peripheral surface of the housing being spaced apart from the drive unit to form a blood flow channel, and the blood flow channel being in communication with the liquid inlet and the liquid outlet, wherein the drive unit comprises a motor and an impeller connected to the motor;
a cannula assembly, the cannula assembly being provided with a liquid discharge port, the cannula assembly comprising a compensation tube, the compensation tube being elastic, and the compensation tube being fixedly connected to and in communication with a distal end of the housing, such that the liquid discharge port is in communication with the liquid outlet of the housing through an inner cavity of the compensation tube; and
a flow straightening device, the flow straightening device being arranged in the blood flow channel, and the flow straightening device being capable of guiding blood to flow from an outer periphery of the motor to the impeller.

12. The blood pump according to claim 11, wherein the motor comprises a motor body and a shaft support base connected to a distal end of the motor body, and the blood flow channel comprises a flow guide section, a flow straightening section and a pressurization section that are sequentially in communication, wherein the flow guide section is located between an outer peripheral surface of the motor body and the inner peripheral surface of the housing, and is in communication with the liquid inlet; the flow straightening section is located between an outer peripheral surface of the shaft support base and the inner peripheral surface of the housing; the pressurization section is located between the impeller and the inner peripheral surface of the housing, and is in communication with the liquid outlet; and the flow straightening device is arranged in the flow straightening section and fixedly connected to the shaft support base.

13. The blood pump according to claim 12, wherein an outer diameter of the shaft support base gradually decreases in a direction from the motor to the impeller, such that the outer peripheral surface of the shaft support base forms a tapered flow guide surface; the flow straightening device comprises a plurality of flow straightening vanes, the plurality of flow straightening vanes are spaced apart on the tapered flow guide surface in a circumferential direction of the shaft support base, and an outer side edge of each flow straightening vane away from the tapered flow guide surface is parallel to a motor shaft.

14. The blood pump according to claim 11, wherein the flow straightening device comprises a plurality of flow straightening vanes, the plurality of flow straightening vanes being fixed on an outer peripheral surface of the motor and spaced apart in a circumferential direction of the motor, and the plurality of flow straightening vanes each extending in an axial direction of the motor, so as to form a linearly extending flow straightening channel between each two adjacent flow straightening vanes.

15. The blood pump according to claim 14, wherein each flow straightening vane comprises a proximal vane body and a distal vane body, the proximal vane body and the distal vane body being arranged in the axial direction of the motor and connected to each other, the distal vane body having a first side edge away from the outer peripheral surface of the motor, the proximal vane body having a second side edge away from the outer peripheral surface of the motor, and at least one of the second side edge or the first side edge being fixedly connected to the inner peripheral surface of the housing.

16. The blood pump according to claim 15, wherein the first side edge is spaced apart from the inner peripheral surface of the housing, and the second side edge is fixedly connected to the inner peripheral surface of the housing, wherein a fourth axial length of the first side edge is greater than a fifth axial length of the second side edge; or, a connection between the first side edge and the second side edge is provided with a rounded corner.

17. The blood pump according to claim 14, wherein the blood pump further has at least one of the following features:
each flow straightening vane has an inflow end face and an outflow end face, the inflow end face and the outflow end face are respectively located at two ends of the flow straightening vane, the inflow end face faces away from the impeller, the outflow end face faces toward the impeller, at least one of the inflow end face or the outflow end face is configured in an arc shape from one vane surface to the other vane surface of the flow straightening vane, and a center of the arc is located on a bisector of a thickness of the flow straightening vane;
the thickness of the flow straightening vane gradually increases in a direction from the motor to the impeller; or
distal portions of the plurality of flow straightening vanes are all curved towards a same side in the circumferential direction of the motor, and a curving direction is consistent with a rotation direction of the impeller.

18. The blood pump according to claim 14, wherein the flow straightening device further comprises a fixing ring, the fixing ring surrounds outer peripheries of the plurality of flow straightening vanes, the fixing ring is fixedly connected to the housing, and at least a part of an inner wall surface of the fixing ring is fixedly connected to outer side edges of the flow straightening vanes to support the housing; and
the housing comprises an inlet tube and a connection tube, and the inlet tube and the connection tube are respectively connected to two ends of the fixing ring and coaxially arranged with the fixing ring, such that the fixing ring constitutes a middle portion of the housing, wherein a proximal end of the inlet tube is provided with the liquid inlet, and a distal end of the connection tube is provided with the liquid outlet.

19. The blood pump according to claim 18, wherein the inlet tube comprises a straight tube, a diameter-variable tube, and a plurality of connection arms connecting the straight tube and the diameter-variable tube;
the straight tube is connected to the fixing ring, inner wall surfaces of the straight tube, the fixing ring and the connection tube are aligned in an axial direction of the straight tube, and outer wall surfaces of the straight tube, the fixing ring and the connection tube are also aligned in the axial direction of the straight tube;
the diameter-variable tube is sleeved over an outer periphery of a connection between the catheter and the drive unit, and is fixedly connected to at least one of the drive unit or the catheter; and
the plurality of connection arms are spaced apart in a circumferential direction of the straight tube, and one liquid inlet is formed between each two adjacent connection arms.

20. The blood pump according to claim 11, wherein a distal end of the blood pump is capable of passing through the right atrium, the right ventricle and the pulmonary valve sequentially to extend into the pulmonary artery, such that the liquid inlet is located in the right ventricle, and the liquid outlet is located in the pulmonary artery; and
the blood pump comprises the catheter, the catheter having a proximal portion, a distal portion and a bending portion, the bending portion being located between the proximal portion and the distal portion and adjacent to the distal portion, and the bending portion being pre-formed into a bent shape, such that a shape of the bending portion matches an anatomical structure of a first communication site or a second communication site of the right atrium, wherein the first communication site is a communication site between the right atrium and the inferior vena cava, and the second communication site is a communication site between the right atrium and the superior vena cava.
